# EUROPEAN PATENT SPECIFICATION

(11) **EP 3 574 085 B1**
(45) Date of publication and mention of the grant of the patent: **08.12.2021**
(21) Application number: 17778308.1
(22) Date of filing: 06.10.2017
(51) Int. Cl.: C12N 1/20, C02F 3/34, A62D 3/02, A62D 101/22, C02F 101/36

(54) **CONSORTIUM OF MICROORGANISMS AND METHOD FOR TREATING BODIES OF WATER CONTAMINATED WITH 1,2-DICHLOROETHANE**
KONSORTIUM VON MIKOROORGANISMEN UND METHODE ZUR BEHANDLUNG VON WASSERMASSEN, DIE MIT 1,2-DICHLOROETHAN KONTAMINIERT SIND.
CONSORTIUM DE MICROORGANISMES ET METHODE DE TRAITEMENT DE MASSES D'EAU CONTAMINÉES PAR LE 1,2-DICHLOROÉTHANE

(30) Priority: 27.01.2017 IT 201700008808
(43) Date of publication of application: 04.12.2019
(73) Proprietor: Eni Rewind S.p.A., 20097 San Donato Milanese (MI) (IT)
(72) Inventor: CARPANI, Giovanna, I-26010 Sergnano (CR) (IT); DE FERRA, Francesca, I-26900 Lodi (IT)
(74) Representative: Cernuzzi, Daniele
(86) International application number: PCT/EP2017/075468
(87) International publication number: WO 2018/137798

(56) References cited:
- EP-A2- 1 352 977
- JP-B2- 5 764 880
- A. GROSTERN ET AL: "Characterization of a Dehalobacter Coculture That Dechlorinates 1,2-Dichloroethane to Ethene and Identification of the Putative Reductive Dehalogenase Gene", APPLIED AND ENVIRONMENTAL MICROBIOLOGY, vol. 75, no. 9, 1 May 2009 (2009-05-01), pages 2684-2693, XP055391505, ISSN: 0099-2240, DOI: 10.1128/AEM.02037-08
- MARZORATI MASSIMO ET AL: "Bacterial diversity and reductive dehalogenase redundancy in a 1,2-dichloroethane-degrading bacterial consortium enriched from a contaminated aquifer", MICROBIAL CELL FACTORIES, BIOMED CENTRAL, GB, vol. 9, no. 1, 19 February 2010 (2010-02-19), page 12, XP021067682, ISSN: 1475-2859
- GIUSEPPE MERLINO ET AL: "Diverse Reductive Dehalogenases Are Associated with Clostridiales-Enriched Microcosms Dechlorinating 1,2-Dichloroethane", BIOMED RESEARCH INTERNATIONAL, vol. 2015, 1 January 2015 (2015-01-01), pages 1-11, XP055391100, ISSN: 2314-6133, DOI: 10.1155/2015/242856

## Description

The present invention relates to the sector of environmental safety and more precisely the sector of environmental remediation.

In particular, the invention relates to a consortium of microorganisms useful for the bioremediation of bodies of water contaminated with 1,2-dichloroethane (1,2-DCA).

Even more preferably, the present invention relates to a consortium of microorganisms that can degrade 1,2-DCA to ethylene, substantially without producing toxic halogenated intermediates, said consortium, when present in an aqueous liquid, preferably under anaerobic conditions, being tolerant to a concentration of 1,2-DCA in solution in said aqueous liquid greater than or equal to 1000 mg/L, preferably comprised between 1000 mg/L and 4000 mg/L, and more preferably comprised between 2000 mg/L and 4000 mg/L. In a second aspect, the present invention relates to a method for the bioremediation of a body of water contaminated by 1,2-DCA, where in said body of water the concentration of 1,2-DCA in solution may be greater than or equal to 1000 mg/L, preferably comprised between 1000 mg/L and 4000 mg/L and more preferably comprised between 2000 mg/L and 4000 mg/L, which comprises placing in contact with said body of water, preferably under anaerobic conditions, the aforementioned consortium of microorganisms able to degrade the 1,2-DCA.

The aforementioned method may be used, for example, for the bioremediation of confined or unconfined aquifers, aquatic sediments, submerged soils, water tables, and may be applied both *on site,* i.e. to the water extracted from said body of water and *in situ,* i.e. directly to the body of water, and preferably it is performed *in situ.*

1,2-DCA is harmful to humans and animals, is an irritant to the respiratory tracts, highly flammable, and is a possible carcinogenic agent (group 2B of the *International Agency for Research on Cancer,* IARC).

The main use of 1,2-DCA is in the plastic industry, as an intermediate in the synthesis of vinyl chloride, in turn a precursor of polyvinyl chloride (PVC). Accidental spills and the negligent management of the production, storage, transport and use processes in the aforementioned industrial sector, have contributed to the spread of significant quantities of this hazardous pollutant in the environment. In particular, the water solubility and high chemical stability (the half-life in anaerobic aquifers is about 50 years) of 1,2-DCA, make it a "perennial" pollutant of aquifers, with a very high risk to the environment and to human health.

Conventionally, soils contaminated with persistent pollutants may be treated *ex situ,* i.e. removed and subjected to chemical/physical treatments such as, for example, washing with solvents, emulsifiers and detergents, vapor at high temperature, etc.

In particular, for the chemical/physical treatment of contaminated bodies of water it is conventional to resort to extraction techniques with solvents, thermal desorption and stripping of pollutants, preferably coupled with adsorption processes on solid matrices (e.g. active carbon, functionalized zeolites, etc.), in order to make the aforementioned pollutants available for chemical or physical degradation. However, these treatments are very expensive and energy intensive, and also pose the problem of the disposal of the contaminated adsorbent materials.

An advantageous alternative is represented by *in situ* bioremediation methods, which exploit the degradation activity of autochthonous and/or exogenous microorganisms, promoting the transformation of toxic organic compounds into harmless substances such as, for example, carbon dioxide, methane, water, inorganic salts and the like.

It is however important to note that the family of toxic organochlorinated compounds persisting in the environment is very extensive and comprises compounds that are very different from one another.

In general, chlorinated hydrocarbons having a higher number of chlorine atoms are generally recalcitrant to aerobic degradation, while they are commonly biotransformed in conditions with a lack of oxygen.

Over recent years, research into dehalogenation by anaerobic microorganisms has been particularly intense. Numerous species of the *Dehalococcoides, Dehalobacter, Desulfitobacterium* and *Dehalogenimonas* genera have been shown to be able to couple the reductive dehalogenation of organohalogenated compounds with the conservation of energy and growth, through a process defined as *OrganoHalide Respiration* (OHR), and for some of these it has been possible to identify the enzymes and isolate the genes involved in this particular metabolic route.

These anaerobic microorganisms, which exploit sources of carbon in the reduced state (e.g. lactate, succinate, pyruvate, benzoate, propionate or formiate) and/or hydrogen as electron donors and are able to use the halogenated compounds as final electron acceptors in anaerobic respiration (Smidt, H. and de Vos, W.M. "Anaerobic microbial dehalogenation" (2004), Annu. Rev. Microbiol., vol. 58, pag. 43-73) are therefore the best candidates for *in situ* bioremediation treatments.

It is necessary to consider that for some species that are particularly recalcitrant to *in vitro* cultures and requiring conditions of total absence of oxygen, the study of enzyme activity associated with reductive dehalogenation has been shown to be particularly challenging and has required the use of innovative "ecogenomics" techniques, as described, for example, in F. Maphosa et al., "Ecogenomics of microbial communities in bioremediation of chlorinated contaminated sites" (2012) Front. Microbiol., vol. 3, art. 351.

It is also important to note that the degradation of organohalogenated compounds may lead to the formation of intermediates that are just as harmful or even more harmful than the starting substrates, (for example trichloroethane (TCE) and tetrachloroethane (PCE) are transformed into vinyl chloride), actually limiting the application of these microorganisms for *in situ* bioremediation.

Despite this, numerous bioremediation methods for aquifers and soils contaminated by chlorinated alkenes or alkanes via microbiological methods are described in the known art.

For example, EP 0 864 542 A2 describes a method for the degradation of chlorinated ethylenes and propylenes present in ground water, which is conveyed, after the addition of an electron donor compound, into a reactor containing a microbial consortium able to degrade halogenated alkenes under anaerobic conditions.

US 6,001,252 on the other hand describes a method for the *in situ* bioremediation of aquifers which comprises the inoculation in the contaminated site of a bacterial consortium able to promote the dehalogenation of the solvents present under anaerobic conditions.

Bioremediation interventions may also comprise the "biostimulation" of degrading microorganisms already present in the contaminated site, providing them with the essential compounds (e.g. nutrients, gases, sources of carbon, mineral salts, etc.) for the purpose of making them prosper and making them able to efficiently perform the task of decomposing the pollutants.

For example, EP 1 600 220 A1 describes an "additive" for use in the bioremediation of soils, water tables or sediments contaminated by organic halides, which comprises a material for developing and activating a microbial consortium comprising active yeast, inactive yeast and yeast extract, a material for promoting reductive dehalogenation by the anaerobic microorganisms, which comprises a compound selected from propionic acid, butyric acid, lactic acid and salts thereof and a material for developing anaerobic conditions, which comprises a compound selected from glucose, galactose, fructose, lactose, sucrose, maltose and starch.

Furthermore, bioremediation strategies may include "bioaugmentation" interventions, through which certain bacterial species with excellent degrading abilities are introduced into contaminated sites, with the aim of supporting or substituting the activity of the autochthonous species when the latter alone are not shown to be able to guarantee a significant bioremediation process in reasonable time frames.

For example, WO 95/32278 describes a method for the bioremediation of soil contaminated by polycyclic aromatic hydrocarbons, comprising the introduction into the soil of a mixed bacterial culture of *Acrhromonas sp.* and *Mycobacterium sp.,* previously accustomed to the polycyclic aromatic hydrocarbons, together with a system of nutrients containing a source of nitrogen and a source of phosphate in order to support the growth of said microorganisms.

In none of the above cases is the bioremediation method applied to sites contaminated by one of the most important and dangerous chlorinated compounds, i.e. 1,2-DCA.

EP 1 352 977 A2 describes a bacterial strain belonging to the *Desulfitobacterium* genus able to dehalogenate dichloroalkanes and trichloroalkanes with high conversion speeds, possibly in the presence of bacterial species able to produce vitamin K, such as, *Enterococcus sp.* It is important to note that the strain described in EP 1 352 977 A2 was isolated from a layer of soil contaminated by 1,2-DCA in a concentration equal to 50 mg/kg and its dehalogenating activity was measured using concentrations of dichloroalkanes and trichloroalkanes no greater than 400 µM.

Similarly, US 8,673,614 describes a microbial consortium comprising strains of the *Clostridium, Acetobacter, Dehalobacter, Bacterioides, Proteobacteria* and *Dehalococcoides* genera, isolated from samples of sediment from a site contaminated by chloroethanes and chloroethylenes in an anaerobic medium containing chloroethane and an electron donor compound selected from lactate, succinate, pyruvate, benzoate, propionate or formiate. Also in this case the concentration of chlorinated organic compounds is never greater than 50 µM.

Finally, patent JP 5764880 B2 describes a strain of *Geobacter sp.* able to transform 1,2-DCA to ethylene through an anaerobic dehalogenation reaction, although not displaying any degradation activity on chlorinated pollutants such as tetrachloroethylene, 1,1,2-trichloroethane, 2,4,6-trichlorophenol, l'hexachlorobenzene. The aforementioned strain of *Geobacter sp.,* whose activity was measured on 1,2-DCA in concentrations no greater than 1 mM, was used in presence of other bacterial species that act on other pollutants (for example, able to dehalogenate vinyl chloride), for the optimization of the bioremediation capacity.

However, the known solutions still have margins for improvement.

In general, many environmental factors, such as the lack of nutritional factors, non-optimal pH and/or redox potential values, the presence or absence of oxygen, may reduce the degrading capacity of the microorganisms. Autochthonous bacterial species or those introduced through bioaugmentation interventions, implied in bioremediation processes for contaminated aquifers may die in presence of chlorinated organic compounds; as described, for example, by J.C. Koenig et al. in "Tolerance of Anaerobic Bacteria to Chlorinated Solvent" (2014), Microbes Environ., vol. 29, pag. 23-30, a significant limitation to the bioremediation of sites contaminated by chlorinated organic compounds actually consists of the onset of toxic effects by said compounds on the microorganisms, especially due to damage to the bacterial membranes connected with the solvent properties for the lipids of these compounds. In this context, the type of chlorinated organic compounds is important, since some of these have been shown to be more recalcitrant to the degradation performed by the microorganisms.

For example, the presence of 1,2-DCA may inhibit the degradation of other pollutants, both halogenated and non-halogenated (as described, for example, by B.A. Kocamemi et al. in "Inhibitory effect for the xenobiotic 1,2-dichloroethane in a nitrifying biofilm reactor" (2007), Water Sci. Technol., vol. 55, pag. 67-73 and by K. Mayer-Blackwell et al., in "1,2-Dichloroethane Exposure Alters the Population Structure, Metabolism, and Kinetics of a Trichloroethene-Dechlorinating Dehalococcoides mccartyi Consortium" (2016), Environ. Sci. Technol., vol. 50, pag. 12187-12196), compromising the efficacy of the bioremediation intervention.

For this reason, most of the reductive dehalogenation studies of 1,2-DCA by microbial consortia have been performed using decisively reduced concentrations of said pollutant (for example, from 0.1 to 0.5 mM), and the most resistant species described in literature have shown dehalogenating activity, although not optimal, up to concentrations equal to 8.7 mM of 1,2-DCA (A.D. Maness et al., "Dehalogenimonas spp. can reductively dehalogenate high concentrations of 1,2-dichloroethane, 1,2-dichloropropane, and 1,1,2-trichloroethane" (2012) AMB Express, vol. 2, art. 54).

However, there are numerous contaminated sites in which the quantities of these contaminants may be substantially higher (for example M. Marzorati et al. in "A novel reductive dehalogenase, identified in a contaminated groundwater enrichment culture and in Desulfitobacterium dichloroeliminans strain DCA1, is linked to dehalogenation of 1,2-dichloroethane" (2007) Appl. Environ. Microbiol. vol. 73, pag. 2990-2999 describes an aquifer contaminated with high concentrations, up to 9 mM, of 1,2-DCA).

There is therefore a strongly felt need to be able to have a method available for treating a body of water contaminated by chlorinated organic compounds, in particular contaminated by 1,2-DCA, in particular when the concentration of this contaminant is high, which can overcome the drawbacks highlighted in methods of the prior art.

The Applicant has now identified a consortium of microorganisms able to promote the degradation of 1,2-DCA to ethylene, preferably under anaerobic conditions, without producing toxic halogenated intermediates, efficiently, said consortium, when present in an aqueous liquid, also being tolerant to high concentrations of said contaminant, unexpectedly significantly higher with respect to the tolerated concentrations for adaptation and typically found in the prior art.

The consortium according to the invention is defined by the attached independent claims 1 and 2.

According to the invention, the aforementioned consortium comprises the microorganisms deposited on 8 November 2016 in accordance with the Budapest Treaty on the "National Collection of Industrial Food and Marine Bacteria" (NCIMB), Aberdeen, Scotland, UK, to which accession number NCIMB 42687 was assigned, as claimed by claim 1; or is derived by such a consortium as claimed by claim 2.

In particular, the degrading activity on the 1,2-DCA substrate by the aforementioned consortium is also displayed, preferably under anaerobic conditions, when such pollutant is present in solution in said aqueous liquid at concentrations greater than or equal to 1000 mg/L and comprised between 1000 mg/L and 4000 mg/L, even more preferably comprised between 2000 mg/L and 4000 mg/L.

The Applicant has also identified a method for the bioremediation of a body of water contaminated by high concentrations of 1,2-DCA in solution, preferably under anaerobic conditions, which uses the aforementioned consortium of microorganisms. Preferably, said method is performed *in situ,* i.e. without it being necessary to extract the water from said body of water.

Thanks to the ability of the aforementioned consortium to maintain the dehalogenating activity, preferably under anaerobic conditions, in presence of high concentrations of 1,2-DCA, preferably at a concentration greater than or equal to 1000 mg/l, more preferably comprised between 1000 mg/l and 4000 mg/l, even more preferably comprised between 2000 mg/l and 4000 mg/l, the method according to the present invention has the advantage of being able to guarantee the bioremediation of a body of water or an aquifer wherein the concentration of said pollutant in solution can reach and exceed the maximum concentration values of said pollutant that can be treated with the bioremediation methods described in the art.

In particular, with the method according to the present invention, it is possible to bioremediate, preferably under anaerobic conditions, a body of water contaminated by 1,2-DCA wherein the concentration of said pollutant in solution may be greater than or equal to 1000 mg/L, preferably comprised between 1000 mg/L and 4000 mg/L, more preferably comprised between 2000 mg/L and 4000 mg/L.

With the method according to the present invention, the body of water contaminated by 1,2-DCA can be treated *in situ*: in other words, the bioremediation of the polluted water may be performed without it having to be taken from the body of water. Despite this, the aforementioned method is also suitable for *on-site* treatments, i.e. on the water extracted from the body of water and placed in contact with the dehalogenating consortium in a second confined space, such as a cistern, a tank, a bath, a reactor, etc. The bioremediation of the body of water with the consortium NCIMB 42687 is preferably performed under anaerobic conditions.

It is important to note that the method according to the present invention may obtain public acceptance without difficulties both due to its beneficial effect on the environment, connected with the dehalogenation activity expressed by the consortium NCIMB 42687 on the pollutant 1,2-DCA, and due to the fact that said consortium does not comprise microorganisms manipulated with genetic engineering techniques.

Further characteristics and advantages of the present invention will become clear from the following detailed description.

For the purposes of the present description and following claims, the definitions of the numeric ranges always include the extremes unless specified otherwise. In the description of the embodiments of the present invention, the use of the terms "comprising" and "containing" indicates that the options described, for example regarding the steps of a method or of a process or the components of a product or of a device, are not necessarily all-inclusive. It is however important to note that the present application also relates to the embodiments in which the term "comprising" in relation to the options described, e.g. regarding the steps of a method or of a process or the components of a product or of a device, must be interpreted as "which essentially consists of" or "which consists of", even if this is not explicitly stated.

For the purposes of the present description and following claims, "ecogenomics" means a range of technologies that do not require the isolation and reproduction of the microorganisms but are applied directly to environmental samples, comprising whole genome amplification, "high-throughput" sequencing of the DNA ("metagenomics") and the RNA ("metatranscriptomics"), the identification of peptides ("metaproteomics") and metabolites ("metabolomics") through mass spectrometry, and other sophisticated techniques that are combined according to new modelling approaches and thanks to computational analysis, in an integrated ecological framework.

For the purposes of the present description and following claims, the term "consortium" of microorganisms means a mixed culture of two or more species of microorganisms, where the two or more species can live in symbiosis and/or be involved at least in part in one or more biological processes where each species can take advantage from the other components of the consortium.

The mixed culture in an ideal case may be syntropic, i.e. characterized by microorganisms all operating in the same biological process, such as the bioremediation of soils, in an ordered and synergistic way towards a single aim, such as, the bioremediation of polluting compounds, and/or syntrophic, i.e. characterized by species of microorganisms that prosper at the expense of the metabolites produced by other species, in an interdependent relationship.

For the purposes of the present description and following claims, the terms "tolerance to 1,2-DCA", or simply "tolerance", used to describe a microorganism or a consortium of microorganisms, means the ability of a microorganism or a consortium of microorganisms to survive and multiply in an aqueous liquid, for example in a liquid or aquiferous medium in which 1,2-DCA is present in a concentration greater than or equal to 1000 mg/L, preferably in a concentration comprised between 1000 mg/L and 4000 mg/L and more preferably in a concentration comprised between 2000 mg/L and 4000 mg/L. When the microorganism is anaerobic or the consortium of microorganisms comprises at least one anaerobic microorganism, the aforementioned "tolerance" is generally preferably displayed when said microorganism or said consortium is kept under anaerobic conditions.

For the purposes of the present description and the following claims, "sequence identity percentage (%)" referring to a nucleotide or amino acid sequence is defined as the percentage of nucleotides or of amino acids in a nucleotide or amino acid sequence being examined that are identical to the nucleotides or to the amino acids of a reference nucleotide or amino acid sequence. The sequence identity can be determined using programs that are known to a person skilled in the art, such as BLAST, ALIGN and CLUSTAL, using standard methods (for example as described by S. F. Altschul, et al. in "Basic Local Alignment Search Tool" (1990), J. Mol. Biol. vol. 215, pag. 403-410, by S. Henikoff et al. in "Amino Acid Substitution Matrices from Protein Blocks" (1992), Proc. Natl. Acad Sci. USA, vol. 89, pag. 10915-10919, by S. Karlin et al. in "Applications and Statistics for Multiple High-Scoring Segments in Molecular Sequences" (1993), Proc. Natl Acad. Sci USA, vol. 90, pag. 5873-5877 and by D.G. Higgins et al. in "CLUSTAL: a Package for Performing Multiple Sequence Alignment on a Microcomputer" (1988), Gene, vol. 73, pag.237-244. The software for performing BLAST analyses is made available to the public by the National Center for Biotechnology Information (NCBI). Furthermore, it is possible to perform research in the sequence databases using the program FASTA (W.R. Pearson et al., "Improved Tools for Biological Sequence Comparison" (1988), Proc. Natl. Acad. Sci. USA, vol. 85, pag. 2444-2448).

An indication that two sequences of amino acids (polypeptides) are substantially identical is given by the immunological cross-reactivity between the two polypeptides. Generally, polypeptides that differ from one another through conservative substitutions of amino acids, i.e. in which one amino acid is substituted with another characterized by a lateral group with similar biochemical properties, have immunological cross-reactivity. Therefore, a polypeptide is substantially identical to a second polypeptide, for example, when the two polypeptides differ from one another by conservative substitution of amino acids. An indication that two nucleotide sequences are substantially identical (they have a sequence identity of 97% or greater than 97%) is that the two molecules (one of which is defined as the "probe") hybridize together under stringent conditions (e.g. in a medium-high "stringency" range). "Hybridization conditions" means the conditions in which the hybridization reactions are performed. These conditions are generally classified based on the degree of "stringency" of the conditions with which the hybridization is measured. The degree of "stringency" can be based, for example, on the difference between the hybridization temperature and the Melting Temperature, Tₘ, of the complex formed by the nucleotide sequence being examined and by the "probe" sequence. Alternatively, or additionally, the degree of stringency can be based on the ionic force conditions of the solutions in which the hybridization experiments are performed.

Functionally, the hybridization of a polynucleotide with a probe module in maximum stringency conditions corresponds to a total or almost total sequence identity between the two sequences.

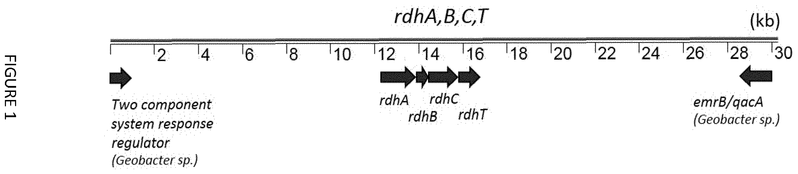

For the purpose of better understanding the characteristics of the process according to the present invention, reference shall also be made to the drawings of the appended figures that have a purely illustrative and absolutely non-limiting function. In particular, Figure 1 schematically represents the genome fragment of *Geobacter sp.* which represents the region of DNA in which the genes associated with the enzyme 1,2-dichloroethane dehalogenase are present. The enzyme activity is typically attributed to the set of proteins codified by the gene *rdhA* and *rdhB.* In the aforementioned DNA region, there are homologous sequences to the *"Two component system response regulator"* (5' end) and to the *emrB*/*qacA* (3' end) genes of *Geobacter sp.*

Figure 2 represents a graph showing the reduction, over nine years, of the concentration of 1,2-DCA in solution in the water contained in a piezometric well placed in an industrial site contaminated by 1,2-DCA, and from which the consortium NCIMB 42687 was isolated.

Figure 3 is a graph showing the percentage reduction of the concentration of 1,2-DCA (Figure 3a) and a graph showing the percentage increase of ethylene production (Figure 3b) in microcosms in which the consortium NCIMB 42687 is cultivated in presence of concentrations of 1,2-DCA equal to 50 mg/L, 300 mg/L, 1000 mg/L, 2000 mg/L and 4000 mg/L. The quantity of ethylene is represented as the area under the peak of the gas chromatogram associated with such compound.

Figure 4 represents a graph showing the reduction of the concentration of 1,2-DCA in the aforementioned piezometric well over the course of the experiment described in Example 4.

It is important to note that the drawings of the appended figures intend to illustrate the general characteristics of the method and/or the materials used in some embodiments of the invention and to complete the written description below.

These drawings do not necessarily accurately reflect the precise structural characteristics or performance of a given embodiment and should not be interpreted as defining or limiting an interval of values or properties of said embodiment.

The present invention relates firstly to a consortium of microorganisms, deposited on 8 November 2016 in accordance with the Budapest Treaty at the "National Collection of Industrial Food and Marine Bacteria" (NCIMB), Aberdeen, Scotland, UK, to which accession number NCIMB 42687 was assigned. The aforementioned consortium NCIMB 42687 can degrade, preferably under anaerobic conditions, the 1,2-DCA to ethylene, substantially without producing toxic halogenated intermediates and can be tolerant to a concentration of 1,2-DCA greater than or equal to 1000 mg/L, preferably comprised between 1000 mg/L and 4000 mg/L, and more preferably comprised between 2000 mg/L and 4000 mg/L.

The concentration of 1,2-DCA relates to the quantity of said contaminant in solution per unit of volume, for example, in an aqueous liquid in which the aforementioned consortium is present and in which it is, for example, maintained or cultivated.

The consortium of the present invention comprises both obligate anaerobic species of microorganisms and oxygen tolerant species. For this reason, all the operations relating to said consortium are performed so as to reduce, as much as possible, contact with the atmospheric oxygen and/or that dissolved in an aqueous liquid.

The aforementioned consortium NCIMB 42687 was isolated starting from samples of water coming from an industrial site polluted by 1,2-DCA, and is characterized by stable and constant reductive dehalogenation activity on the aforementioned chlorinated hydrocarbon and tolerance to 1,2-DCA in particularly high concentrations, as specified above.

The reductive dehalogenation activity characteristic of the NCIMB consortium may be responsible for the degradation of 1,2-DCA by the aforementioned consortium and for the production of ethylene.

The degradation of the 1,2-DCA, and the concomitant production of ethylene, by the consortium NCIMB 42687 may be associated with the reductive dehalogenation activity by at least one 1,2-dichloroethane dehalogenase enzyme synthesized by at least one microbial species of the consortium.

In a preferred aspect, said consortium of microorganisms can synthesize at least one 1,2-dichloroethane dehalogenase enzyme having an amino acid sequence that has a sequence identity equal to 97% or greater than 97%, or greater than 98%, or greater than 99% with the sequence SEQ ID NO: 6. More preferably, said consortium of microorganisms can synthesize at least one 1,2-dichloroethane dehalogenase enzyme having an identical amino acid sequence to the sequence SEQ ID NO: 6.

To determine the identifying characteristics of its components, the consortium NCIMB 42687 was subjected to the analysis of the ribosomal DNA (16S rDNA profiling) through metagenomic sequencing using Ion Torrent^{™} technology, following the protocols provided by the manufacturer, as described in Example 2, through which it was possible to isolate and characterize four different sequences of 16S rDNA, representing the preponderant species of microorganisms in said consortium, which correspond to the sequences SEQ ID NO: 1, SEQ ID NO: 2, SEQ ID NO: 3 and SEQ ID NO:4.

Based on the comparison with the sequences present in the reference databases using the program BLAST^{®} (NCBI), it was possible to attribute the four different sequences of 16S rDNA to the species: *Geobacter sp.* (SEQ ID NO: 1), *Pseudomonas sp.* (SEQ ID NO: *2), Clostridium sp.* (SEQ ID NO: 3) and *Acidovorax sp.* (SEQ ID NO: 4).

In other words, based on the analysis of the 16S ribosomal DNA it is considered that the anaerobic consortium NCIMB 42687 may preponderantly comprise at least one strain of the species *Geobacter sp.,* at least one strain of the species *Pseudomonas sp.,* at least one strain of the species *Clostridium sp.* and at least one strain of the species *Acidovorax sp*. The aforementioned consortium may comprise further species of anaerobic or oxygen-tolerant microorganisms, with respect to those indicated above, which cannot however be identified either through the traditional microbiological techniques of bacterial isolation known to a person skilled in the art or through the metagenomic analyses described above, therefore they are not considered significant for the purposes of the present invention.

Preferably, the consortium may comprise microorganisms characterized by at least one nucleotide sequence that has a sequence identity equal to 97%, or greater than 97%, or greater than 98%, or greater than 99% with the sequence SEQ ID NO: 1, or with the sequence SEQ ID NO: 2, or with the sequence SEQ ID NO: 3, or with the sequence SEQ ID NO: 4, or by combinations of nucleotide sequences, each of which has a sequence identity equal to 97%, or greater than 97%, or greater than 98%, or greater than 99% with the sequence SEQ ID NO:1, or with the sequence SEQ ID NO: 2, or with the sequence SEQ ID NO: 3, or with the sequence SEQ ID NO: 4.

In a particularly preferred aspect the consortium of microorganisms NCIMB 42687 may comprise at least one strain of at least one species of *Geobacter sp.* having an identical 16S rDNA nucleotide sequence to the nucleotide sequence SEQ ID NO: 1, at least one strain of at least one species of *Pseudomonas sp.* having an identical 16S rDNA nucleotide sequence to the nucleotide sequence SEQ ID NO: 2, at least one strain of at least one species of *Clostridium sp.* having an identical 16S rDNA nucleotide sequence to the nucleotide sequence SEQ ID NO: 3, at least one strain of at least one species of *Acidovorax sp.* having an identical 16S rDNA nucleotide sequence to the nucleotide sequence SEQ ID NO: 4.

From the analyses of the genomic DNA extracted from samples of the consortium NCIMB 42687 it can further be demonstrated that the reductive dehalogenation activity displayed by the aforementioned consortium is connected with the abundance of 16S rDNA homologous to the sequence SEQ ID NO: 1. For this reason, it is reasonable to suppose that the enzyme responsible for such reductive dehalogenation activity is synthesized by at least one strain of the species associated with the sequence SEQ ID NO: 1, i.e.

*Geobacter sp.* This hypothesis is confirmed by the result of the sequencing of the metagenome of the consortium, as described better below.

Therefore, in a preferred aspect, the consortium NCIMB 42687 comprises at least one strain of at least one species of *Geobacter sp.* having a 16S rDNA nucleotide sequence that may have a sequence identity equal to 97%, or greater than 97%, or greater than 98%, or greater than 99%, or is identical to the nucleotide sequence SEQ ID NO: 1, in whose genomic DNA there may be at least one *rdhA* gene coding for the 1,2-dichloroethane dehalogenase enzyme.

Preferably, said *rdhA* gene coding for the 1,2-dichloroethane dehalogenase enzyme may have a sequence identity equal to 97%, or greater than 97%, or greater than 98%, or greater than 99% with the sequence SEQ ID NO: 5. Preferably, said *rdhA* gene has an identical nucleotide sequence to the sequence SEQ ID NO: 5.

Preferably, said *rdhA* gene codes for a protein of 551 amino acids that may have a sequence identity equal to 97%, or greater than 97%, or greater than 98%, or greater than 99% with the sequence SEQ ID NO: 6. Preferably, said *rdhA* gene codes for a protein having an identical amino acid sequence to the sequence SEQ ID NO: 6.

The metagenomic sequencing using Ion Torrent^{™} technology and following the protocols provided by the manufacturer starting from samples of said anaerobic consortium NCIMB 42687 has demonstrated that the aforementioned *rdhA* gene is present in a region of about 4000 pairs of bases, which may comprise four genes that have a high homology with the four genes *rdhA, rdhB, rdhC* and *rdhT,* described, for example, by Futagami T., Goto M., Furukawa K. in "Biochemical and genetic bases of dehalorespiration" (2008) Chem Record, vol. 8, pag. 1-12.

Said region of genomic DNA is represented schematically in Figure 1.

The analysis of the nucleotide sequences both upstream and downstream of the aforementioned genomic DNA of about 4000 pairs of bases has enabled sequences to be identified which, from the analysis of the homology with sequences contained in the databases consulted appear to be characteristic of the species *Geobacter sp.* and univocally attributable to such species. This confirms the hypothesis that the *rdhA* gene may be present in the genomic DNA of the species *Geobacter sp.*

In a preferred aspect of the invention, the aforementioned *rdhA* gene may be comprised in a genomic DNA region that can comprise a sequence attributable to the *rdhB* gene, which codes for an anchoring protein to the membrane associated with the dehalogenating enzyme, having a sequence that can have a sequence identity equal to 97%, or greater than 97%, or greater than 98%, or greater than 99% with the sequence SEQ ID NO: 7 and more preferably may have an identical sequence to the sequence SEQ ID NO: 7.

In a preferred aspect of the invention, the aforementioned *rdhA* gene may be comprised in a genomic DNA region that may comprise a sequence attributable to the *rdhC* gene, coding for a protein probably with a transcriptional regulation function of the *rhdA* gene, having a sequence that may have a sequence identity equal to 97%, or greater than 97%, or greater than 98%, or greater than 99% with the sequence SEQ ID NO: 8 and more preferably may have an identical sequence to the sequence SEQ ID NO: 8.

In a preferred aspect of the invention, the aforementioned *rdhA* gene may be comprised in a genomic DNA region that may comprise a sequence attributable to the *rdhT* gene, coding for a protein probably involved in the correct folding in the presence of a corrinoid factor of the precursor of the dehalogenating enzyme, having a sequence that may have a sequence identity equal to 97%, or greater than 97%, or greater than 98%, or greater than 99% with the sequence SEQ ID NO: 9 and more preferably may have an identical sequence to the sequence SEQ ID NO: 9.

Preferably, the *rdhA* gene may be present in the genomic DNA of the *Geobacter sp.* species in combination with at least one of the sequences having sequence identities equal to 97%, or greater than 97%, or greater than 98%, or greater than 99%, or identical to the sequences SEQ ID NO: 7, SEQ ID NO: 8 and SEQ ID NO: 9.

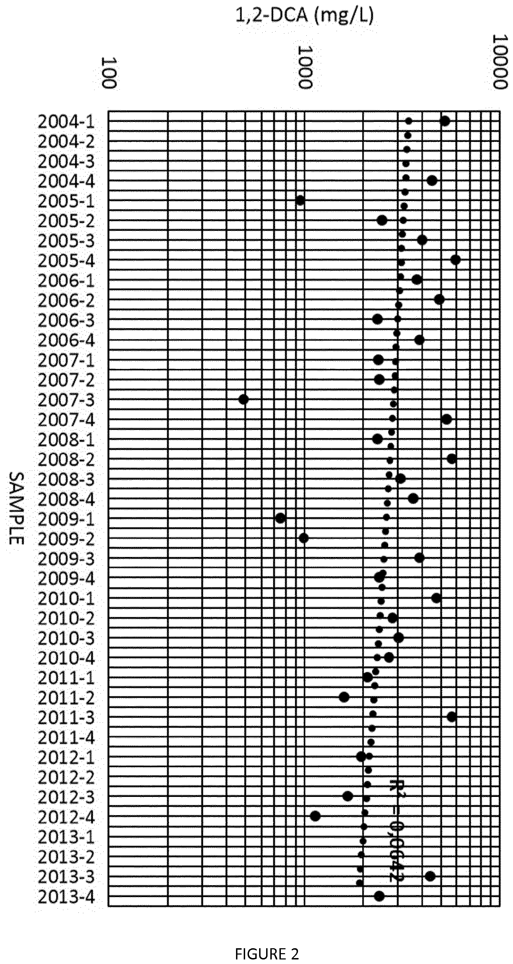

As mentioned above, the consortium NCIMB 42687 was isolated from samples of water taken from a piezometric well placed in the aforementioned industrial site at the end of a monitoring campaign of the concentration of 1,2-DCA, performed from 2004 until 2013. The data related to the concentration of 1,2-DCA in solution measured during the aforementioned monitoring campaign are shown in the graph of Figure 2. In particular, it is to be noted that the concentration of pollutant varies over time. Despite a certain fluctuation being highlighted in the dispersion of the data, typical of an unconfined system and connected with phenomena of spreading from and towards the surrounding soil, from the trend line it is possible to demonstrate that the concentration of 1,2-DCA in solution is sensitively reduced over time, showing that the consortium effectively displays degradation activity against 1,2-DCA *in situ.*

The evidence that such degradation activity is associated with the consortium according to the present invention was provided by the results of the analysis of the microorganisms present in samples taken during the aforementioned campaign through extraction of genomic DNA, amplification and sequencing of fragments separated with *Denaturing Gradient Gel Electrophoresis* (DGGE) techniques. Since 2005, when the first analyses of this type were performed, the composition of bacterial species has been kept constant and in particular it has always been possible to demonstrate in the water samples coming from the aforementioned piezometric well the presence of microorganisms of the *Geobacter sp.* species.

Through experiments in microcosms, the consortium NCIMB 42687 has been shown to be effectively able to degrade the 1,2-DCA to ethylene substantially without producing toxic halogenated intermediates, preferably under anaerobic conditions, and is therefore particularly suitable for the bioremediation of bodies of water contaminated by said chlorinated hydrocarbon.

In particular, said consortium can degrade the 1,2-DCA through reductive dehalogenation even when said pollutant is present in particularly high concentrations in solution and, in fact, it can be maintained in culture in microcosms comprising an aqueous liquid in which 1,2-DCA is present in solution in a concentration greater than or equal to 1000 mg/L, preferably comprised between 1000 mg/L and 4000 mg/L and more preferably comprised between 2000 mg/L and 4000 mg/L, as it also appears to be vital, able to perform dehalogenation activity, and can therefore degrade the 1,2-DCA in bodies of water in which the concentration of 1,2-DCA in solution may be greater than or equal to 4000 mg/L, as is clear in Figure 2.

Since within the consortium NCIMB 42687 a species that expresses dehalogenating enzyme activity at high concentrations of 1,2-DCA is *Geobacter sp.,* it follows that said species of *Geobacter sp.* present in said consortium is at least one of the species that are tolerant to 1,2-DCA.

In other words, the consortium NCIMB 42687 is characterized by the fact that it comprises at least one strain of a species of *Geobacter sp.,* which displays dehalogenating activity on 1,2-DCA and which may be tolerant to a concentration of said 1,2-DCA in solution greater than or equal to 1000 mg/L, preferably comprised between 1000 mg/L and 4000 mg/L and more preferably comprised between 2000 mg/L and 4000 mg/L.

The consortium NCIMB 42687 may preferably be maintained or cultivated in a synthetic medium, preferably under anaerobic conditions. The aforementioned synthetic medium may be liquid or solid and is preferably liquid.

Therefore, in a preferred aspect, the present invention relates to a culture of said consortium in a synthetic medium, preferably liquid synthetic medium and preferably under anaerobic conditions. Preferably, said synthetic medium comprises at least one electron donor compound, for example an organic acid or a salt thereof, an alcohol, a sugar, or another substrate based on carbon, which simulates the dehalogenation activity of the microorganisms of the consortium and promotes the establishment of anaerobiosis. Preferably, said at least one electron donor compound is selected from the group comprising lactic acid, succinic acid, pyruvic acid, benzoic acid, propionic acid, formic acid and salts thereof with an alkaline metal or alkaline earth metal. In a particularly preferred aspect, said electron donor compound is sodium lactate.

The present invention secondly relates to a method for the bioremediation of a body of water contaminated by 1,2-DCA, where in said body of water the concentration of 1,2-DCA in solution in said body of water may be greater than or equal to 1000 mg/L, preferably comprised between 1000 mg/L and 4000 mg/L and more preferably comprised between 2000 mg/L and 4000 mg/L, which comprises placing in contact with said body of water the consortium NCIMB 42687, preferably under anaerobic conditions.

Preferably, said body of water may comprise, in addition to the 1,2-DCA, one or more halogenated aromatic or aliphatic hydrocarbons in solution, for example tetrachloroethylene (PCE) or trichloroethylene (TCE).

In a preferred aspect, the microorganisms of the consortium NCIMB 42687 used in the bioremediation method according to the invention may be in the immobilized form, in accordance with the technologies known to a person skilled in the art. For example, the microorganisms can be immobilized through adhesion to organic or inorganic supports, such as activated carbon or glass, or through incorporation in natural or artificial polymer matrices, for example agarose, gelatin, cellulose, alginate, polyurethane, polyester, etc. In a preferred aspect, the aforementioned method for the bioremediation of a body of water contaminated by 1,2-DCA according to the present invention, preferably performed under anaerobic conditions, may comprise the steps of:
a) providing a culture of the enzyme NCIMB 42687 in a synthetic medium containing 1,2-DCA in a concentration greater than or equal to 1000 mg/L, preferably comprised between 1000 mg/L and 4000 mg/L, more preferably comprised between 2000 mg/L and 4000 mg/L;
b) placing said culture of the consortium NCIMB 42687 obtained in step a) in contact with the aforementioned body of water.

Step a) of the present method may be performed by growing the aforementioned consortium in any receptacle, for example, microcosm bottles, bioreactors, columns filled with synthetic medium, in accordance with the methods known to a person skilled in the art in which it is possible to recreate an anaerobic environment.

Preferably, the culture of the consortium is created using a synthetic medium based on mineral salts, such as the minimal medium BTZ-3 described by E.R. Hendrickson et al. in "Molecular Analysis of Dehalococcoides 16S Ribosomal DNA from Chloroethene-Contaminated Sites throughout North America and Europe" (2002), Appl. Envir*on.*

*Microbiol.,* vol. 68, pag. 485-495, enriched with cysteine, vitamins, and trace metal salts, having a pH comprised between 6.5 and 7.5, as described in Example 1.

Preferably, the culture is performed under anaerobic conditions. To create the anaerobic condition, the culture medium may be subjected to degassing and/or insufflation of inert gases, such as nitrogen, helium, argon, and preferably nitrogen.

In a preferred aspect, in the synthetic medium used for the preparation of the consortium culture of step a) at least one electron donor compound may also be comprised, for example an organic acid or a salt thereof, an alcohol, a sugar, or another substrate based on carbon, which simulates the dehalogenation activity of the microorganisms and promotes the establishment of anaerobiosis.

More preferably, said at least one electron donor compound is selected from the group comprising lactic acid, succinic acid, pyruvic acid, benzoic acid, propionic acid, formic acid and salts thereof with an alkaline metal or alkaline earth metal. In a particularly preferred aspect, said electron donor compound is sodium lactate.

Preferably, said culture provided in step a) is created at a temperature comprised between 15°C and 30°C and more preferably at a temperature comprised between 20°C and 26°C. The duration of step a) may depend on the volume of synthetic medium used and the quantity of microorganisms belonging to the desired consortium, and generally is such as to allow a culture to be obtained in which said microorganisms of the consortium are in the exponential or late exponential phase of growth.

Step b) of the present method may be performed in different ways, according to whether the treatment is conducted *in situ* or *on site.* For example, said step b) may be conducted by pumping or injecting the culture obtained in step a) directly into the body of water contaminated by 1,2-DCA, through one or more injection points.

The methods with which the consortium culture is placed in contact with the body of water contaminated by 1,2-DCA may be similar, but not limited, to those described in the prior art, for example, in "Demonstration of Bioaugmentation at Kelly AFB, Texas (2007), ESTCP Cost and Performance Report Environmental Security Technology Certification Program, U.S. Department of Defence (ER-9914), or as described by C.E. Aziz, R.A.Wymore, R.J.Steffan in "Bioaugmentation - Considerations in Bioaugmentation for Groundwater Remediation" (2013) H.F.Stroo, A. Leeson, C.H.Ward Editors, Springer New York Press.

The volume of culture to be used in step b) may be determined by a person skilled in the art on an individual basis, so as to obtain in the body of water a sufficient quantity of microorganisms of the consortium to guarantee that the consortium itself is kept alive and its propagation. Generally, the volume of culture used is such as not to determine the substantial dilution of the pollutant in the body of water. Furthermore, the ratio between the volume of water to be treated and the volume of culture may increase or decrease based on the growth rate of the consortium in the culture obtained in step a) and the characteristics of the water to be treated, such as its pH, the level of nutrients and electron donor compounds and the concentration of dissolved oxygen. When the method is performed *in situ,* the aforementioned ratio may also depend on the groundwater flow, the nature and degree of permeability of the soil, and all the other factors that may affect the capacity of the culture of microorganisms to be distributed uniformly and to propagate in the body of water.

As the results of the campaign described above, and presented in Figure 2, show, when the quantity of microorganisms of the consortium used in step b) is adequate, the species of microorganisms of said consortium multiply at the expense of the nutrients present in the body of water and maintain the property of degrading the 1,2-DCA for a very long time, without it being necessary to add a further volume of said culture in order to guarantee the bioremediation effect.

However, the degradation speed of the pollutant could be too low with respect to the desired one.

To speed up the bioremediation process of the body of water, in a preferred aspect of the invention, the method described above could comprise a further step c):
c) feeding said body of water with an aqueous solution, optionally buffered to a pH comprised between 6.0 and 8.0, comprising at least one electron donor compound.

As underlined above, the electron donor compound has the function of stimulating the reductive dehalogenation activity and promoting the maintenance of the anaerobic conditions.

In the method according to the present invention, any electron donor compound can be used, selected, for example, from organic acids and salts thereof, sugars, alcohols, or other carbon-based substrates, which stimulate the activity of the microorganisms and promote the establishment of anaerobiosis.

In a preferred aspect, said at least one electron donor compound is used in a form that promotes its slow release in the body of water (for example, as a compound in polymer form such as polylactate).

In a preferred aspect, said at least one electron donor compound is a salt of an alkaline metal or alkaline earth metal of an organic acid selected from the group comprising lactic acid, succinic acid, pyruvic acid, benzoic acid, propionic acid, formic acid and mixtures thereof and more preferably said electron donor compound is sodium lactate. Preferably, said at least one electron donor compound is added to the body of water in quantities such as to obtain a final concentration in said body of water comprised between 0.1 mM and 5 mM, and preferably comprised between 1 mM and 2 mM.

Any buffer system known to a person skilled in the art may be used for optionally buffering the aqueous solution fed to said body of water in step c) of the method according to the invention. The function of the buffer system is also that of correcting any pH excesses in the water present in the body of water to be treated with the consortium according to the present invention.

Preferably, said aqueous solution is buffered with a phosphate buffer system at pH comprised between 6.5 and 8.0, more preferably at pH comprised between 7.0 and 7.5. Preferably said buffer system is present in said aqueous solution in concentrations comprised between 1 mM and 500 mM, more preferably between 10 mM and 250 mM. The aqueous solution of said electron donor compound, optionally buffered, may be treated prior to use for the purpose of removing the oxygen contained therein, for example through degassing, and/or insufflation of inert gases, such as nitrogen, helium and argon. However, it is important to note that the additional step c) of the method in accordance with the invention may also be performed by adding to the body of water said aqueous solution comprising said electron donor compound, optionally buffered, without removing the oxygen dissolved therein, without any perturbation being observed in the dehalogenating activity performed by the consortium NCIMB 42687 placed in contact with the body of water.

It is therefore clear that, although it comprises anaerobic microorganisms, the consortium NCIMB 42687 (and in particular the species that expresses the dehalogenation enzyme activity, i.e. *Geobacter sp*.) can withstand temporary exposure to moderate quantities of dissolved oxygen without detrimental effects to the enzyme activity or to the survival of the microorganisms.

However, in the case in which the body of water treated is particularly rich in dissolved oxygen, it is possible to reduce the concentration of said dissolved oxygen, for example through the insufflation into said body of water of inert gases such as nitrogen, helium or argon, or through the use of antioxidant chemical agents (so called "oxygen scavengers") or through the use of cultures of obligated anaerobic microorganisms, in presence of appropriate nutrients.

The degree of anaerobiosis can be determined by measuring the concentration of dissolved oxygen and the potential redox of the body of water, through electrochemical measurements, for example with multiparameter probes, in accordance with standard methods known to a person skilled in the art, such as, ASTM D1498 - 14 and ASTM D888 - 12.

The method for the bioremediation of a body of water contaminated by 1,2-DCA, in accordance with the present invention, preferably performed *in situ,* represents an advantageous alternative to the bioremediation of said body of water by autochthonous dehalogenating microorganisms, in particular when the aforementioned autochthonous dehalogenating microorganisms are completely absent or are present at ineffective concentrations for the purpose of bioremediation, even after stimulation with appropriate electron donor compounds, as described above, or when the concentration of 1,2-DCA in the body of water exceeds a threshold value, preferably greater than or equal to 1000 mg/L, beyond which inhibiting or toxic effects are displayed on the aforementioned autochthonous microorganisms.

The degradation speed of the 1,2-DCA by the consortium NCIMB 42687 depends on the initial concentration of the contaminant and can be determined through gas chromatography analysis on the headspace of samples taken directly from the microcosms. Preferably, the speed is comprised between 5 and 100 ppm of 1,2-DCA degraded/day, more preferably comprised between 5 and 200 ppm of 1,2-DCA degraded/day, even more preferably between 6 and 10 ppm of 1,2-DCA degraded/day. Example 4 describes a bioremediation test on an aquifer contaminated with 1,2-DCA and in which the bioremediation efficacy by the consortium NCIMB 42687 is demonstrated, after the addition of an aqueous solution of an electron donor compound, in accordance with step c) of the method according to the present invention.

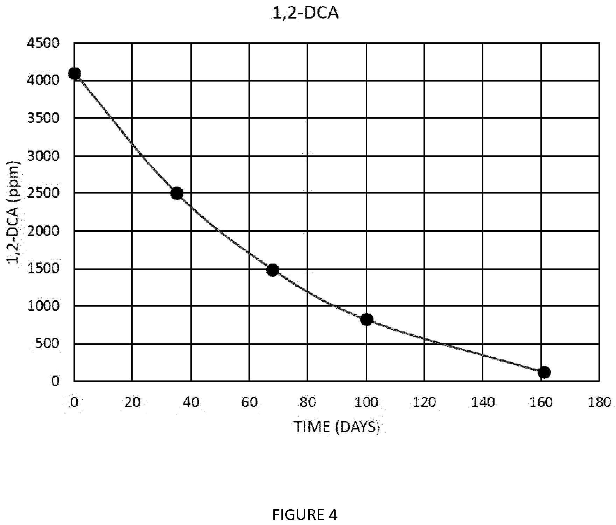

The results of this experiment are shown in Figure 4.

Sampling performed at time intervals of about 30 days in a period of about 160 days on the aforementioned aquifer from which the consortium NCIMB 42687 was isolated to which an electron donor compound was supplied, have enabled the efficacy of said electron donor compound in substantially increasing the degradation speed of 1,2-DCA by the consortium itself to be confirmed.

On the samples taken over this period of time, an analysis of the metagenomic DNA was also performed, which confirmed the co-presence over time of the species of microorganisms of the consortium NCIMB 42687.

Furthermore, with the aim of excluding the effect of an apparent reduction of the pollutant connected with scouring phenomena, the effective degradation of the 1,2-DCA by the dehalogenating activity associated with the consortium NCIMB 42687, was demonstrated by subjecting the samples taken from the aquifer to "Compound Specific Isotopic Analysis" (CSIA): the isotopic analyses (Solid Phase Microextraction - Gas Chromatography - Isotope Ratio Mass Spectrometry, SPM-GC-IRMS) were performed as described by M. Schmidt et al., in "Comparison of 1,2-dichloroethane, dichloroethene and vinyl chloride carbon stable isotope fractionation during dechlorination by two Dehalococcoides strains" (2014), Water Research, vol. 52, pag. 146-154, using CO₂ as the reference gas, on a Delta V^{™} device made by ThermoFisher Scientific.

Said analyses highlighted a significant variation over time in the isotropic composition (δ¹³C (‰)), connected with an increase in the isotopic ratio ¹³C/¹²C in the molecules of 1,2-DCA present in the samples taken from the aquifer. Since the enzymes, in particular the 1,2-DCA dehalogenase enzyme, have a preference for substrates containing the isotope ¹²C, evidence of the increase in the isotropic ratio ¹³C/¹²C shows that the 1,2-DCA is effectively subject to enzyme degradation by the consortium according to the present invention.

In a preferred aspect, the aforementioned method may be used for the bioremediation of an aquifer contaminated by 1,2-DCA in which there are substantially no autochthonous dehalogenating microorganisms present.

As already mentioned, the method according to the present invention can be performed both *in situ* and *on site* and is preferably performed *in situ.* Any contaminated body of water may be treated with the consortium according to the present invention. In particular, the consortium NCIMB 42687 may be placed in contact with bodies of water, for example: ground water, natural and/or artificial basin water, waste water of industrial origin, percolating water from landfill, waste water of domestic origin, etc. contaminated by 1,2-DCA, preferably in concentration greater than or equal to 1000 mg/L, more preferably comprised between 1000 and 4000 mg/L, even more preferably comprised between 2000 and 4000 mg/L, in order to degrade the 1,2-DCA itself.

For the purpose of putting the present invention into practice and illustrating it more clearly, below are some non-limitative examples.

### Example 1 (Isolation of the consortium of microorganisms NCIMB 42687)

From a piezometric well placed inside an area of about 250 m² present in an industrial site and characterized by "chronic" pollution by 1,2-DCA, ten 1000 mL samples of water were taken, subsequently insufflated with gaseous N₂ for 16 hours. From the aforementioned samples, 20 microcosms were prepared in an anaerobic hood (each sample represented in double), taking 98 mL of water from each 1000 mL sample, which were split into two sterile 55 mL bottles containing: 0.5 mL of "BTZ-3 100x" medium (NH₄Cl 4.3 mg/L, KH₂PO₄ 50 mg/L, MgCl₂·6H₂O 20 mg/L, CaCl₃·2H₂O 1 mg/L and buffer HEPES 50 µM, brought to pH 7.5 with NaOH), cysteine hydrochloride (4 mg/mL), 0.4 mL/L of "Trace Metals Solution 200x" (Nitrilotriacetic acid 12.8 g/L, FeCl₃·6H₂O 1,35 g/L, MnCl₆·4H₂O 0,1 g/L, COCl₂·6H₂O 24 mg/L, CaCl₂·2H₂O 0,1 g/L, ZnCl₂ 0,1 g/L, CuCl₂·2H₂O 25 mg/L, H₃BO₃ 10 mg/L, Na₂MoO₄·2H₂O 24 mg/L, NaCl 1 g/L, NiCl₂·6H₂O 0,12 g/L, Na₂SeO₃·2H₂O 26 mg/L), 50 µL of "Seven Vitamins Solution 1000x" (cobalamin 100 mg/L, p-aminobenzoic acid 80 mg/L, D(+)-biotin 20 mg/L, nicotinic acid 200 mg/L, calcium pantothenate 100 mg/L, pyridoxine-HCI 300 mg/L, thiamine-HCl·2H₂O 200 mg/L), and 100 µL of an aqueous solution of 1 M sodium lactate. Prior to use, all the solutions were sterilized by filtration and made anaerobic through insufflation of N₂ for 10 minutes. At the end, 1 mL of a sterile aqueous solution of 1,2-DCA 5000 mg/L was added. The total volume of liquid in the microcosm bottles therefore amounts to about 51.05 mL.

Subsequently, the bottles, maintained under anaerobic conditions, were closed with a Teflon^{®} cap and sealed with an aluminum ring. The microcosms were kept at 26°C without agitation and in the dark.

After 7 days a 1mL sample was taken from each microcosm on which headspace gas chromatography analysis was performed.

For the analysis, 1 mL of culture of each microcosm was placed in a 20 mL glass vial containing 9 mL of distilled water and 4 g of NaCl. Each sample was then incubated at 80°C for 20 minutes under moderate agitation. After that 1 µL taken from the headspace was injected into gas chromatography equipped with a DB-5MS UI column (Agilent Technologies, 60 m, 0.25 mm ID, 0.25 µm film).

All the cultures highlighted comparable degradation activity on the 1,2-DCA present in the medium. One of these was subjected to some refreshment steps with sterile medium prepared as described above and a tolerance test to the 1,2-DCA was performed on the microcosm obtained.

To do this, from the microcosm in question, five microcosms in duplicate were prepared through refreshment, as described above with the difference that the 1,2-DCA was added in the five microcosms at respective final concentrations of 50 mg/L, 300 mg/L, 1000 mg/L, 2000 mg/L and 4000 mg/L. Also in this case, the microcosms were kept at 26°C without agitation and in the dark. On pre-fixed days, a sample was taken from each microcosm on which headspace gas chromatography analysis was performed as described above.

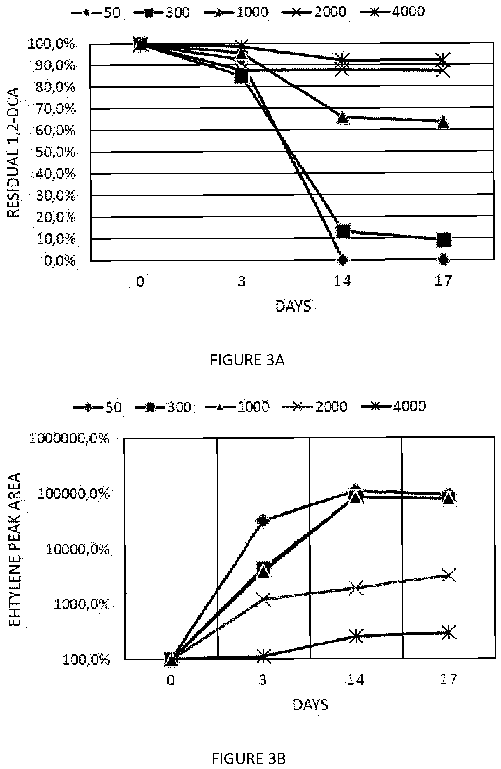

Figure 3 shows a graph of the percentage variations corresponding to the reduction in concentration of 1,2-DCA (Figure 3a) and the increase in concentration of ethylene (Figure 3b) measured after 3, 14 and 17 days of growth of the culture in the different microcosms characterized by increasing concentrations of 1,2-DCA.

The consortium of microorganisms of the aforementioned culture was deposited at the "National Collection of Industrial Food and Marine Bacteria" (NCIMB) in Aberdeen, Scotland, UK, and the filing number NCIMB 42687 was assigned thereto.

### Example 2 (Sequencing of the metagenome of the consortium of microorganisms NCIMB 42687 and identification of the 16S rDNA)

The isolation of the genomic DNA of the microorganisms that constitute the consortium NCIMB 42687 was performed with the kit "FastDNA^{®} SPIN Kit for Soil" (MP Biomedicals) following the instructions contained in the protocol "Ion Torrent Plus gDNA Fragment library preparation User guide 4471989 revD" of the manufacturer Life Technologies.

In particular, the DNA was fragmented enzymatically with the "Ion Shear Plus Reagents Kit" used in accordance with the instructions provided by the manufacturer. The DNA thus treated was bound to the P1 adapters and bar-coded adapter 2 in accordance with the protocol mentioned above and therefore subjected to agarose gel electrophoresis. The fragments with an average length around 300-400 bp were eluted by the agarose gel and purified. The DNA of three "bar coded libraries" of fragments obtained as described above was quantified using the BioAnalyzer tool according to the manufacturer's protocol. Thereafter the bar coded libraries were mixed following the protocol itself and subjected to sequencing on Ion 316 chip (v2) following the instructions contained in the "IonTorrent Ion PGM Sequencing 400 kit" manual (Publ number MAN0007242).

The sequences of DNA obtained were compared with sequences of DNA contained in the non-redundant "Bacteria" database using the software BLASTN^{®} (NCBI, U.S. National Library of Medicine) and aligned with sequences of 16S bacterial ribosomal DNA through the program "Local Blast" (BioEdit) using the file of total sequences (over 1,8·10⁶ bases) as the "Local Nucleotide Database File".

In this way it was possible to identify four different sequences of 16S rDNA, corresponding to the four SEQ ID NO sequences: 1, SEQ ID NO: 2, SEQ ID NO: 3 and SEQ ID NO: 4, which are homologous to sequences corresponding to the preponderant species of microorganisms of the anaerobic consortium NCIMB 42687, i.e. *Geobacter sp., Pseudomonas sp., Clostridium sp.* and *Acidovorax sp.*

In particular, in the set of sequences corresponding to the composition of the consortium from whose culture the metagenomic DNA analyzed was extracted, a quarter of the total sequences with a coverage index equal to 6,2x can be attributed to rDNA 16 S sequences of *Geobacter sp.*

### Example 3 (Identification of the genomic DNA which comprises the genes correlated to 1,2-dichloroethane dehalogenases of Geobacter sp.).

Starting from the file of total sequences constituting the "Local Nucleotide Database File", obtained as described in Example 2 above, and using as a reference the sequences of genes correlated with the dehalogenase of *Desulfitobacterium dichloroeliminans* (described by M. Marzorati, et al. in "A novel reductive dehalogenase, identified in a contaminated groundwater enrichment culture and in Desulfitobacterium dichloroeliminans strain DCA1, is linked to dehalogenation of 1,2-dichloroethane" (2007) Applied and Environ. Microbiol., vol. 73, pag. 2990-2999), through the program "Local Blast" (BioEdit) nucleotide sequences have been identified corresponding to the genes *rdhA, rdhB, rdhC* and *rdhT* in a region of DNA of about 4000 pairs of bases, which is flanked by characteristic sequences of *Geobacter sp.*

The aforementioned region of DNA is represented schematically in Figure 1. The sequences of the four genes are identified with the SEQ ID NO codes: 5, SEQ ID NO: 6, SEQ ID NO: 7, SEQ ID NO: 8 and SEQ ID NO: 9.

### Example 4 (Bioremediation test in situ in an aquifer with the consortium NCIMB 42687)

For the purpose of verifying the efficacy of the consortium NCIMB 42687 *in situ,* the variation in the concentration of 1,2-DCA in water contained in the piezometric well mentioned in Example 1 was measured, i.e. in the same aquifer from which the consortium was isolated, after stimulation of the dehalogenation activity of said consortium with an electron donor compound.

For that purpose, 7000 L of an aqueous solution of sodium lactate were prepared, obtained by diluting in water 450 kg of a commercial solution of 60% sodium lactate by weight in water.

The aqueous solution of sodium lactate was equally split and pumped into four injection wells especially created in proximity to the aforementioned piezometric well and comprised in the 250 m² area already described in Example 1.

At prefixed intervals of time, a sample of water was taken from the aforementioned piezometric well, that was analyzed to measure the variation in concentration of 1,2-DCA through gas chromatography, as described in Example 1.

The results of the gas chromatography analysis are reported in Table 1 and presented in Figure 4.

As per the results, the concentration of 1,2-DCA in solution in the water in the piezometric well in which the consortium NCIMB 42687 is present stimulated with sodium lactate as the electron donor passes from over 4000 mg/L to 122 mg/L (variation: -97%).

**Table 1: variation in the concentration of 1,2-DCA in solution**

| Sampling time (days) | Concentration of 1,2-DCA (mg/L) |
|---|---|
| 0 | 4100 |
| 35 | 2500 |
| 68 | 1490 |
| 100 | 820 |
| 161 | 122 |

The average degradation speed is seen to be about 10 times higher than the degradation speed observed prior to the treatment.

Starting from the samples taken, microcosms were prepared and maintained from which the metagenomic DNA was extracted and sequenced. In all the samples it was possible to confirm the persistence of the consortium NCIMB 42687.

Finally, it is however to be understood that further changes and variations may be made to the process described and illustrated herein which do not depart from the scope of the appended claims.

## Claims

1. Consortium of microorganisms deposited under accession number NCIMB 42687 which degrades, preferably under anaerobic conditions, 1,2-dichloroethane to ethylene, said consortium being tolerant to a concentration of 1,2-dichloroethane greater than or equal to 1000 mg/L, preferably comprised between 1000 mg/L and 4000 mg/L, and more preferably comprised between 2000 mg/L and 4000 mg/L.

2. Consortium of microorganisms derived from the consortium of microorganism deposited under accession number NCIMB 42687 and which maintains the ability to degrade, preferably under anaerobic conditions, 1,2-dichloroethane to ethylene, said consortium being tolerant to a concentration of 1,2-dichloroethane greater than or equal to 1000 mg/L, preferably comprised between 1000 mg/L and 4000 mg/L, and more preferably comprised between 2000 mg/L and 4000 mg/L, said consortium comprising at least one strain of at least one species of *Geobacter sp.* having a 16S rDNA nucleotide sequence having greater than 99% identity with SEQ ID NO: 1, at least one strain of at least one species of *Pseudomonas sp.* having a 16S rDNA nucleotide sequence having greater than 99% identity with SEQ ID NO: 2, at least one strain of at least one species of *Clostridium sp.* having a 16S rDNA nucleotide sequence having greater than 99% identity with SEQ ID NO: 3, and at least one strain of at least one species of *Acidovorax sp.* having a 16S rDNA nucleotide sequence having greater than 99% identity with SEQ ID NO: 4.

3. Consortium of microorganisms according to claim 1 or 2, which synthesizes at least one 1,2-dichloroethane dehalogenase enzyme having an identical amino acid sequence to the sequence SEQ ID NO: 6.

4. Consortium of microorganisms according to any one of claims 1 to 3, comprising at least one strain of at least one species of *Geobacter sp*. having an identical 16S rDNA nucleotide sequence to the nucleotide sequence SEQ ID NO: 1, at least one strain of at least one species of *Pseudomonas sp.* having an identical 16S rDNA nucleotide sequence to the nucleotide sequence SEQ ID NO: 2, at least one strain of at least one species of *Clostridium sp.* having an identical 16S rDNA nucleotide sequence to the nucleotide sequence SEQ ID NO: 3, at least one strain of at least one species of *Acidovorax sp.* having an identical 16S rDNA nucleotide sequence to the nucleotide sequence SEQ ID NO: 4.

5. Consortium of microorganisms according to any one of claims 1 to 4, comprising at least one strain of at least one species of *Geobacter sp.* and in whose genomic DNA there is at least one rdhA gene coding for the 1,2-dichloroethane dehalogenase enzyme.

6. Consortium of microorganisms according to claim 5, wherein said rdhA gene has an identical nucleotide sequence to the sequence SEQ ID NO: 5.

7. Consortium of microorganisms according to claim 6, wherein said rdhA gene codes for a protein having an identical amino acid sequence to the sequence SEQ ID NO: 6.

8. Consortium of microorganisms according to any one of claims 5 to 7, wherein said rdhA gene is comprised in a genomic DNA region that comprises a sequence that can be attributed to the rdhB gene, having a sequence identical to the sequence SEQ ID NO: 7.

9. Consortium of microorganisms according to any one of claims 5 to 8, wherein said rdhA gene is comprised in a genomic DNA region that comprises a sequence that can be attributed to the rdhC gene, having a sequence identical to the sequence SEQ ID NO: 8.

10. Consortium of microorganisms according to any one of claims 5 to 9, wherein said rdhA gene is comprised in a genomic DNA region that comprises a sequence that can be attributed to the rdhT gene, having an identical-sequence to the sequence SEQ ID NO: 9.

11. Method for the bioremediation of a body of water contaminated by 1,2-dichloroethane, where in said body of water the concentration of 1,2-dichloroethane in solution is greater than or equal to 1000 mg/L, preferably comprised between 1000 mg/L and 4000 mg/L and more preferably comprised between 2000 mg/L and 4000 mg/L, which comprises placing in contact with said body of water the consortium, according to any of claims 1 to 10, preferably under anaerobic conditions.

12. Method according to claim 11, wherein said body of water comprises, in addition to the 1,2-dichloroethane, one or more halogenated aromatic or aliphatic hydrocarbons in solution.

13. Method according to claim 11 or 12, preferably performed under anaerobic conditions, which comprises the steps of:
a) providing a culture of the consortium NCIMB 42687 in a synthetic medium containing 1,2-dichloroethane in a concentration greater than or equal to 1000 mg/L, preferably comprised between 1000 mg/L and 4000 mg/L, more preferably comprised between 2000 mg/L and 4000 mg/L;
b) placing said culture of the consortium NCIMB 42687 obtained in step a) in contact with said body of water.

14. Method according to claim 13, wherein said synthetic medium comprises at least one electron donor compound.

15. Method according to claim 14, wherein said at least one electron donor compound is selected from the group comprising lactic acid, succinic acid, pyruvic acid, benzoic acid, propionic acid, formic acid and salts thereof with an alkaline metal or alkaline earth metal and is preferably sodium lactate.

16. Method according to any one of claims 13 to 15 wherein said culture provided in step a) is created at a temperature comprised between 15°C and 30°C and preferably at a temperature comprised between 20°C and 26°C.

17. Method according to any one of claims 13 to 16, comprising the additional step:
c) feeding said body of water with an aqueous solution, optionally buffered to a pH comprised between 6.0 and 8.0, comprising at least one electron donor compound.

18. Method according to claim 17, wherein said at least one electron donor compound is a salt of an alkaline metal or alkaline earth metal of an organic acid selected from the group comprising lactic acid, succinic acid, pyruvic acid, benzoic acid, propionic acid, formic acid and mixtures thereof and preferably is sodium lactate.

19. Method according to any one of claims 17 to 18, wherein said at least one electron donor compound is added to the body of water in quantities such as to obtain a final concentration in said body of water comprised between 0.1 mM and 5 mM, and preferably comprised between 1 mM and 2 mM.

20. Method according to any one of claims 17 to 19, wherein said aqueous solution is buffered with a phosphate buffer system at pH comprised between 6.5 and 8.0, preferably at pH comprised between 7.0 and 7.5.

21. Method according to any one of claims 11 to 20 where said method is performed in situ.

## Patentansprüche

1. Konsortium von Mikroorganismen, hinterlegt unter der Zugangsnummer NCIMB 42687, das, vorzugsweise unter anaeroben Bedingungen, 1,2-Dichlorethan zu Ethylen abbaut, wobei das Konsortium gegenüber einer Konzentration von 1,2-Dichlorethan von mehr als oder gleich 1000 mg/L, vorzugsweise zwischen 1000 mg/L und 4000 mg/L und mehr bevorzugt zwischen 2000 mg/L und 4000 mg/L, tolerant ist.

2. Konsortium von Mikroorganismen, das von dem unter der Zugangsnummer NCIMB 42687 hinterlegten Konsortium von Mikroorganismen abgeleitet ist und das die Fähigkeit beibehält, vorzugsweise unter anaeroben Bedingungen, 1,2-Dichlorethan zu Ethylen abzubauen, wobei das Konsortium gegenüber einer Konzentration von 1,2-Dichlorethan von mehr als oder gleich 1000 mg/L, vorzugsweise zwischen 1000 mg/L und 4000 mg/L und mehr bevorzugt zwischen 2000 mg/L und 4000 mg/L, tolerant ist, wobei das Konsortium mindestens einen Stamm von mindestens einer Spezies von *Geobacter sp.,* welcher eine 16S rDNA-Nukleotidsequenz aufweist, die mehr als 99% Identität mit SEQ ID NO: 1 aufweist, mindestens einen Stamm von mindestens einer Spezies von *Pseudomonas sp.,* welcher eine 16S rDNA-Nukleotidsequenz aufweist, die mehr als 99% Identität mit SEQ ID NO: 2 aufweist, mindestens einen Stamm von mindestens einer Spezies von *Clostridium sp.,* welcher eine 16S rDNA-Nukleotidsequenz aufweist, die mehr als 99% Identität mit SEQ ID NO: 3 aufweist, und mindestens ein Stamm von mindestens einer Spezies von *Acidovorax sp.,* welcher eine 16S rDNA-Nukleotidsequenz aufweist, die mehr als 99% Identität mit SEQ ID NO: 4 aufweist, umfasst.

3. Konsortium von Mikroorganismen nach Anspruch 1 oder 2, das mindestens ein 1,2-Dichlorethan-Dehalogenase-Enzym, das eine identische Aminosäuresequenz zur Sequenz SEQ ID NO: 6 aufweist, synthetisiert.

4. Konsortium von Mikroorganismen nach einem der Ansprüche 1 bis 3, umfassend mindestens einen Stamm von mindestens einer Spezies von *Geobacter sp.,* welcher eine identische 16S rDNA-Nukleotidsequenz zur Nukleotidsequenz SEQ ID NO: 1 aufweist, mindestens einen Stamm von mindestens einer Spezies von *Pseudomonas sp.,* welcher eine identische 16S rDNA-Nukleotidsequenz zur Nukleotidsequenz SEQ ID NO: 2 aufweist, mindestens einen Stamm von mindestens einer Spezies von *Clostridium sp.,* welcher eine identische 16S rDNA-Nukleotidsequenz zur Nukleotidsequenz SEQ ID NO: 3 aufweist, mindestens ein Stamm von mindestens einer Spezies von *Acidovorax sp.,* welcher eine identische 16S rDNA-Nukleotidsequenz zur Nukleotidsequenz SEQ ID NO: 4 aufweist.

5. Konsortium von Mikroorganismen nach einem der Ansprüche 1 bis 4, umfassend mindestens einen Stamm von mindestens einer Spezies von *Geobacter sp.* und in dessen genomischer DNA mindestens ein rdhA-Gen vorhanden ist, welches das 1,2-Dichlorethan-Dehalogenase-Enzym kodiert.

6. Konsortium von Mikroorganismen nach Anspruch 5, wobei das rdhA-Gen eine identische Nukleotidsequenz zu der Sequenz SEQ ID NO: 5 aufweist.

7. Konsortium von Mikroorganismen nach Anspruch 6, wobei das rdhA-Gen ein Protein kodiert, das eine identische Aminosäuresequenz zu der Sequenz SEQ ID NO: 6 aufweist.

8. Konsortium von Mikroorganismen nach einem der Ansprüche 5 bis 7, wobei das rdhA-Gen in einer genomischen DNA-Region umfasst ist, die eine Sequenz umfasst, welche dem rdhB-Gen zugeordnet werden kann, das eine Sequenz aufweist, die identisch zu der Sequenz SEQ ID NO: 7 ist.

9. Konsortium von Mikroorganismen nach einem der Ansprüche 5 bis 8, wobei das rdhA-Gen in einer genomischen DNA-Region umfasst ist, die eine Sequenz umfasst, welche dem rdhC-Gen zugeordnet werden kann, das eine Sequenz aufweist, die identisch zu der Sequenz SEQ ID NO: 8 ist.

10. Konsortium von Mikroorganismen nach einem der Ansprüche 5 bis 9, wobei das rdhA-Gen in einer genomischen DNA-Region umfasst ist, die eine Sequenz umfasst, die dem rdhT-Gen zugeordnet werden kann, das eine Sequenz aufweist, die identische zu der Sequenz SEQ ID NO: 9 ist.

11. Verfahren zur Bioremediation eines durch 1,2-Dichlorethan kontaminierten Gewässers, wobei in dem Gewässer die Konzentration von 1,2-Dichlorethan in Lösung größer oder gleich 1000 mg/L, vorzugsweise zwischen 1000 mg/L und 4000 mg/L und mehr bevorzugt zwischen 2000 mg/L und 4000 mg/L, ist, welches das Inkontaktbringen des Konsortiums nach einem der Ansprüche 1 bis 10 mit dem Gewässer, vorzugsweise unter anaeroben Bedingungen, umfasst.

12. Verfahren nach Anspruch 11, wobei das Gewässer zusätzlich zu dem 1,2-Dichlorethan einen oder mehrere halogenierte aromatische oder aliphatische Kohlenwasserstoffe in Lösung umfasst.

13. Verfahren nach Anspruch 11 oder 12, vorzugsweise durchgeführt unter anaeroben Bedingungen, das die Schritte umfasst:
a) Bereitstellen einer Kultur des Konsortiums NCIMB 42687 in einem synthetischen Medium, das 1,2-Dichlorethan in einer Konzentration von mehr als oder gleich 1000 mg/L, vorzugsweise zwischen 1000 mg/L und 4000 mg/L, mehr bevorzugt zwischen 2000 mg/L und 4000 mg/L, enthält;
b) Inkontaktbringen der in Schritt a) erhaltenen Kultur des Konsortiums NCIMB 42687 mit dem Gewässer.

14. Verfahren nach Anspruch 13, wobei das synthetische Medium mindestens eine Elektronendonorverbindung umfasst.

15. Verfahren nach Anspruch 14, wobei die mindestens eine Elektronendonorverbindung ausgewählt ist aus der Gruppe, umfassend Milchsäure, Bernsteinsäure, Brenztraubensäure, Benzoesäure, Propionsäure, Ameisensäure und Salze davon mit einem Alkalimetall oder Erdalkalimetall umfasst, und vorzugsweise Natriumlactat ist.

16. Verfahren nach einem der Ansprüche 13 bis 15, wobei die in Schritt a) bereitgestellte Kultur bei einer Temperatur zwischen 15°C und 30°C und vorzugsweise bei einer Temperatur zwischen 20°C und 26°C erzeugt wird.

17. Verfahren nach einem der Ansprüche 13 bis 16, umfassend den zusätzlichen Schritt:
c) Beschicken des Gewässers mit einer wässrigen Lösung, die gegebenenfalls auf einen pH-Wert zwischen 6,0 und 8,0 gepuffert ist, und mindestens eine Elektronendonorverbindung umfasst.

18. Verfahren nach Anspruch 17, wobei die mindestens eine Elektronendonorverbindung ein Salz eines Alkalimetalls oder Erdalkalimetalls einer organischen Säure ist, ausgewählt aus der Gruppe, umfassend Milchsäure, Bernsteinsäure, Brenztraubensäure, Benzoesäure, Propionsäure, Ameisensäure und Mischungen davon, und vorzugsweise Natriumlactat ist.

19. Verfahren nach einem der Ansprüche 17 bis 18, wobei die mindestens eine Elektronendonorverbindung dem Gewässer in solchen Mengen zugesetzt wird, dass eine Endkonzentration in dem Gewässer zwischen 0,1 mM und 5 mM, und vorzugsweise zwischen 1 mM und 2 mM, erhalten wird.

20. Verfahren nach einem der Ansprüche 17 bis 19, wobei die wässrige Lösung mit einem Phosphatpuffersystem bei einem pH-Wert zwischen 6,5 und 8,0, vorzugsweise bei einem pH-Wert zwischen 7,0 und 7,5, gepuffert wird.

21. Verfahren nach einem der Ansprüche 11 bis 20, wobei das Verfahren *in situ* durchgeführt wird.

## Revendications

1. Consortium de microorganismes déposé avec le numéro d'accès NCIMB 42687 qui dégrade, de préférence dans des conditions anaérobies, le 1,2-dichloroéthane en éthylène, ledit consortium étant tolérant à une concentration de 1,2-dichloroéthane supérieure ou égale à 1000 mg/l, de préférence comprise entre 1000 mg/l et 4000 mg/l, et de manière plus préférée comprise entre 2000 mg/l et 4000 mg/l.

2. Consortium de microorganismes dérivé du consortium de microorganismes déposé avec le numéro d'accès NCIMB 42687 et qui conserve la capacité à dégrader, de préférence dans des conditions anaérobies, le 1,2-dichloroéthane en éthylène, ledit consortium étant tolérant à une concentration de 1,2-dichloroéthane supérieure ou égale à 1000 mg/l, de préférence comprise entre 1000 mg/l et 4000 mg/l, et de manière plus préférée comprise entre 2000 mg/l et 4000 mg/l, ledit consortium comprenant au moins une souche d'au moins une espèce de *Geobacter sp.* ayant une séquence nucléotidique de l'ADNr 16S ayant une identité supérieure à 99 % avec SEQ ID NO: 1, au moins une souche d'au moins une espèce de *Pseudomonas sp.* ayant une séquence nucléotidique de l'ADNr 16S ayant une identité supérieure à 99 % avec SEQ ID NO: 2, au moins une souche d'au moins une espèce de *Clostridium sp.* ayant une séquence nucléotidique de l'ADNr 16S ayant une identité supérieure à 99 % avec SEQ ID NO: 3, et au moins une souche d'au moins une espèce d'*Acidovorax sp.* ayant une séquence nucléotidique de l'ADNr 16S ayant une identité supérieure à 99 % avec SEQ ID NO: 4.

3. Consortium de microorganismes selon la revendication 1 ou 2, qui synthétise au moins une enzyme 1,2-dichloroéthane déshalogénase ayant une séquence en acides aminés identique à la séquence SEQ ID NO: 6.

4. Consortium de microorganismes selon l'une quelconque des revendications 1 à 3, comprenant au moins une souche d'au moins une espèce de *Geobacter sp.* ayant une séquence nucléotidique de l'ADNr 16S identique à la séquence nucléotidique SEQ ID NO: 1, au moins une souche d'au moins une espèce de *Pseudomonas sp.* ayant une séquence nucléotidique de l'ADNr 16S identique à la séquence nucléotidique SEQ ID NO: 2, au moins une souche d'au moins une espèce de *Clostridium sp.* ayant une séquence nucléotidique de l'ADNr 16S identique à la séquence nucléotidique SEQ ID NO: 3, et au moins une souche d'au moins une espèce d'*Acidovorax sp.* ayant une séquence nucléotidique de l'ADNr 16S identique à la séquence nucléotidique SEQ ID NO: 4.

5. Consortium de microorganismes selon l'une quelconque des revendications 1 à 4, comprenant au moins une souche d'au moins une espèce de *Geobacter sp.* et dans l'ADN génomique de laquelle il y a au moins un gène rdhA codant l'enzyme 1,2-dichloroéthane déshalogénase.

6. Consortium de microorganismes selon la revendication 5, dans lequel ledit gène rdhA a une séquence nucléotidique identique à la séquence SEQ ID NO: 5.

7. Consortium de microorganismes selon la revendication 6, dans lequel ledit gène rdhA code une protéine ayant une séquence en acides aminés identique à la séquence SEQ ID NO: 6.

8. Consortium de microorganismes selon l'une quelconque des revendications 5 à 7, dans lequel ledit gène rdhA est compris dans une région d'ADN génomique qui comprend une séquence qui peut être attribuée au gène rdhB, ayant une séquence identique à la séquence SEQ ID NO: 7.

9. Consortium de microorganismes selon l'une quelconque des revendications 5 à 8, dans lequel ledit gène rdhA est compris dans une région d'ADN génomique qui comprend une séquence qui peut être attribuée au gène rdhC, ayant une séquence identique à la séquence SEQ ID NO: 8.

10. Consortium de microorganismes selon l'une quelconque des revendications 5 à 9, dans lequel ledit gène rdhA est compris dans une région d'ADN génomique qui comprend une séquence qui peut être attribuée au gène rdhT, ayant une séquence identique à la séquence SEQ ID NO: 9.

11. Procédé de bioréhabilitation d'une masse d'eau contaminée par du 1,2-dichloroéthane, dans lequel, dans ladite masse d'eau, la concentration de 1,2-dichloroéthane en solution est supérieure ou égale à 1000 mg/l, de préférence comprise entre 1000 mg/l et 4000 mg/l et de manière plus préférée comprise entre 2000 mg/l et 4000 mg/l, qui comprend la mise en contact avec ladite masse d'eau du consortium selon l'une quelconque des revendications 1 à 10, de préférence dans des conditions anaérobies.

12. Procédé selon la revendication 11, dans lequel ladite masse d'eau comprend, en plus du 1,2-dichloroéthane, un ou plusieurs hydrocarbures aliphatiques ou aromatiques halogénés en solution.

13. Procédé selon la revendication 11 ou 12, de préférence mis en œuvre dans des conditions anaérobies, qui comprend les étapes suivantes :
a) obtention d'une culture du consortium NCIMB 42687 dans un milieu synthétique contenant du 1,2-dichloroéthane à une concentration supérieure ou égale à 1000 mg/l, de préférence comprise entre 1000 mg/l et 4000 mg/l et de manière encore plus préférée comprise entre 2000 mg/l et 4000 mg/l ;
b) mise en contact de ladite culture du consortium NCIMB 42687 obtenue dans l'étape a) avec ladite masse d'eau.

14. Procédé selon la revendication 13, dans lequel ledit milieu de synthèse comprend au moins un composé donneur d'électrons.

15. Procédé selon la revendication 14, dans lequel ledit au moins un composé donneur d'électrons est choisi dans le groupe comprenant l'acide lactique, l'acide succinique, l'acide pyruvique, l'acide benzoïque, l'acide propionique, l'acide formique et leurs sels avec un métal alcalin ou un métal alcalino-terreux, et est de préférence le lactate de sodium.

16. Procédé selon l'une quelconque des revendications 13 à 15, dans lequel ladite culture obtenue dans l'étape a) est créée à une température comprise entre 15°C et 30°C et de préférence à une température comprise entre 20°C et 26°C.

17. Procédé selon l'une quelconque des revendications 13 à 16, comprenant l'étape additionnelle suivante :
c) introduction dans ladite masse d'eau d'une solution aqueuse, éventuellement tamponnée à un pH compris entre 6,0 et 8,0, comprenant au moins un composé donneur d'électrons.

18. Procédé selon la revendication 17, dans lequel ledit au moins un composé donneur d'électrons est un sel d'un métal alcalin ou d'un métal alcalino-terreux d'un acide organique choisi dans le groupe comprenant l'acide lactique, l'acide succinique, l'acide pyruvique, l'acide benzoïque, l'acide propionique, l'acide formique et leurs mélanges, et de préférence est le lactate de sodium.

19. Procédé selon l'une quelconque des revendications 17 et 18, dans lequel ledit au moins un composé donneur d'électrons est ajouté à la masse d'eau en des quantités telles que soit obtenue une concentration finale dans ladite masse d'eau comprise entre 0,1 mM et 5 mM, et de préférence comprise entre 1 mM et 2 mM.

20. Procédé selon l'une quelconque des revendications 17 à 19, dans lequel ladite solution aqueuse est tamponnée avec un système tampon phosphate à un pH compris entre 6,5 et 8,0, de préférence à un pH compris entre 7,0 et 7,5.

21. Procédé selon l'une quelconque des revendications 11 à 20, lequel procédé est mis en œuvre in situ.


### SEQUENCE LISTING

<110> Syndial servizi ambientali spa
<120> Consortium of microorganisms and method for treating bodies of water
   contaminated with 1,2-dichloroethane
<130> H16017
<160> 9
<170> BiSSAP 1.3.6
<210> 1
   <211> 1834
   <212> DNA
   <213> Geobacter
<220>
   <223> 16S rDNA
<400> 1
   gaaaggaggt gatccagccg caggttcccc tacggctacc ttgttacgac ttcaccccag      60
   tcaccgacca ttccttagga cgctgcctcc cacaaggggt tagctcacgc atttcgggac     120
   caatcgactc ccgtggtgtg acgggcggtg tgtacaaggc ccgggaacgt attcaccgcg     180
   gcatgctgat ccgcgattac tagcgattcc aacttcatag agtcgagttg cagactctaa     240
   tccgaactga gaccggcttt atgagattgg ctccacctcg cggtatcgca actctttgta     300
   ccggccattg tagcacgtgt gtagccctgg tcataagggc catgaggact tgacgtcatc     360
   cccaccttcc tccggtttaa caccggcagt ctccacagag tgcccaactt aatgatggca     420
   actgaggata ggggttgcgc tcgttgcggg acttaaccca acatctcacg acacgagctg     480
   acgacagcca tgcagcacct gtcttacggc tccccgaaag gcacacctcg ctttcacgaa     540
   gcttccgtag atgtcaagac caggtaaggt tctgcgcgtt gcgtcgaatt aaaccacatg     600
   ctccaccgct tgtgcgggcc cccgtcaatt cctttgagtt ttagtcttgc gaccgtactt     660
   cccaggcgga gtacttaatg cgttagctgc ggcactgcag gggtcaatac ccgcaacacc     720
   tagtactcat cgtttacggc gtggactacc agggtatcta atcctgtttg ctacccacgc     780
   tttcgcgtct cagcgtcaat atcggtccag gtagccgcct tcgccaccgg tgttcctcct     840
   aatatctacg gatttcactc ctacactagg aattccacta ccctctcccg tattcaagtc     900
   tgacagtatc caatgcactt cctgggttga gcccagggct ttcacatcag actaatcaga     960
   ccgcctacac gcgctttacg cccaataatt ccgaacaacg cttgcacccc ccgtattacc    1020
   gcggctgctg gcacggagtt agccggtgct tcctttaggg gtaccgtcaa gtataaaggg    1080
   tattagccca aatacatttc tttcccctcg acagagcttt acgacccgaa agccttcatc    1140
   actcacgcgg cgttgctgcg tcaggctttc gcccattgcg caaaattccc cactgctgcc    1200
   tcccgtagga gtctggaccg tgtctcagtt ccagtgtggc tgatcatcct ctcagaccag    1260
   ctaaccatcg tagccttggt aggccattac cctaccaact agctaatggt acgcagactc    1320
   atctgatgac agaaggcccg aaggtccccc ccttttcccg cagagtccaa agactccgtg    1380
   ggcttatccg gtattagcac ctctttcgaa gtgttatccc agatcatcag gcagattatc    1440
   tacgcgttac tcacccgtgc gccactaaat gaaggagcaa gccccttcat tccgttcgac    1500
   ttgcatgtgt taggcacgcc gccagcgttc gttctgagcc aggatcaaac tctccagttt    1560
   attatgaaag tctgattact ttaaactcaa aaatttactt ctggcacttc tcacaattca    1620
   cgttaatcac tgctattcgg ttttcaaaga ccagcgccgc aaccaggcaa aaacctgcgc    1680
   tgcaaccaga cgaacaacat actcaaatca ctcttcgctg tcaacaacta atttcccgac    1740
   aacaatcttt tttctgagca ccctcaaaaa actcccgcct caaaagggac tcgctttcta    1800
   tcataagcgc tttacttatg tcaaaagaaa aatg                                1834
<210> 2
   <211> 996
   <212> DNA
   <213> Pseudomonas
<220>
   <223> 16s rDNA
<400> 2
   ggccttgaag gcgcgcacca cgatgggcac gtcggcatgg ccgctgacga agatcaccgg 60
   caggtcgatg ccgcgcgcca gcagttcgtc ctgcacgttc agcccgccca tgccgggcat 120
   ccgcacgtcg agcaggacgc aggcatggcg gccgccctcg caggcatcga ggaagtcccg 180
   gccctgggtg aagggccgtg ccttgaggcc gaccgattcc agcagccaga ccgtcgattc 240
   gagcatgccc tggtcgtcgt cgaccacgta gaccagcgct tcgtcaggca tagccatcgt 300
   taccctcccg tggcgccggc ggcgagcggc agccggcagt tcatgagcaa cccgccatcc 360
   gccggcagct cggcttcgag ggtgccgccg aagccctcga cgatgctgcg gctcatcgac 420
   aggcccaggc ccagcccttc cggcttgctg gtgtagaacg gcgtgaaaat gtgttccagc 480
   tgatccgccg gcactcccgg tccctggtcg cgaacccaca ggcagagcag cccgccgggc 540
   tcgacgcggc taccgatgac gacccgcgac ggcgagccgg gatgcgcctc gcggttggct 600
   tcgatggcgt tgcgcagcag attgaggaac acctgttcga gcagcacgcg gtcggcatac 660
   agcggcggcg gattgtcggt catcgcttct tccaccgcca cttgccagtg cccggcttcc 720
   cacgcgcaga ggccgatggc ctcgcgtgcc acctggttga ggtcgagcgc ctgcaggcgc 780
   cgctggccct tgcgggatta gataccctgg tagtccacgc cgtaaacgat gtcaactagc 840
   cgttgggagc cttgagctct tagtggcgca gctaacgcat taagttgacc gcctggggag 900
   tacggccgca aggttaaaac tcaaatgaat tgacgggggg cccgcacaag cggtggagca 960
   tgtggtttaa ttcgaagcaa cgcgaagaac cttacc 996
<210> 3
   <211> 1583
   <212> DNA
   <213> Clostridium
<220>
   <223> 16S rDNA
<400> 3
   tttcaaaatt aaacagaatt aaggataaat ttttccagtc tatgaaagct gtgagctttc      60
   atttctccct agaaaggagg tgatccagcc gcaggttctc ctacggctac cttgttacga     120
   cttcacccca atcaccaatc ccaccttcga ctgctggctc cttgcggtta cctcacaggc     180
   ttcgggtgtt accggctctc atggtgtgac gggcggtgtg tacaagaccc gggaacgtat     240
   tcaccgcgac attctgattc gcgattacta gcaactccaa cttcatgtag gcgagtttca     300
   gcctacaatc cgaactggga ccggtttttt agtttggctc cccctcacgg gtttgcttct     360
   cgttgtaccg gccattgtag cacgtgtgta gccctagaca taaggggcat gatgatttga     420
   cgtcatcccc accttcctcc tggttaaccc aggcagtctc gctagagtgc tcaacttaat     480
   ggtagcaact aacgataagg gttgcgctcg ttgcgggact taacccaaca tctcacgaca     540
   cgagctgacg acaaccatgc accacctgtc ttcctgcccc gaagggcttc ctctgttacg     600
   agtaattcag gagatgtcaa gtctaggtaa ggttcttcgc gttgcttcga attaaaccac     660
   atgctccgct gcttgtgcgg gtccccgtca attcctttga gttttaatct tgcgaccgta     720
   ctccccaggc ggaatactta ttgcgtttgc tgcggcaccg agggtggtac cccccgacac     780
   ctagtattca tcgtttacgg cgtggactac cagggtatct aatcctgttt gctccccacg     840
   ctttcgtatc tcagcgtcag ttacggtcca gaaagtcgcc ttcgccactg gtgttcttcc     900
   taatctctac gcatttcacc gctacactag gaattccact ttcctctccc gcactctaga     960
   tacccagttt caaatgcagc gcccaggtta agcccgggaa tttcacatct gacttaaata    1020
   tccgcctaca tactctttac gcccagtaaa tccggacaac gcttgccacc tacgtattac    1080
   cgcggctgct ggcacgtagt tagccgtggc ttcctccttg ggtaccgtca ttatcgtccc    1140
   caaagacaga gctttacaat ccgaagacct tcatcactca cgcggcgttg ctgcgtcagg    1200
   gtttccccca ttgcgcaata ttccccactg ctgcctcccg taggagtctg gaccgtgtct    1260
   cagttccaat gtggccgatc accctctcag gtcggctacg catcgtcgcc ttggtgagcc    1320
   gttacctcac caactagcta atgcgccgcg ggtccatctc aaagcggatt gctcctttga    1380
   ttatcttctc atgcgagaaa ataatgttat gcggtattaa tctccctttc gggaggctat    1440
   ccccctcttt gaggcaggtt acccacgtgt tactcacccg tccgccgcta gaatctcccc    1500
   gaagggatca tctcgctcga cttgcatgtg ttaggcacgc cgccagcgtt cgtcctgagc    1560
   caggatcaaa ctctcaattt aaa                                            1583
<210> 4
   <211> 1609
   <212> DNA
   <213> Acidovorax
<220>
   <223> 16S rDNA
<400> 4
   aaatttatca agatcgaact gtagagtttg atcctggctc agattgaacg ctggcggcat      60
   gccttacaca tgcaagtcga acggtaacag gtcttcggat gctgacgagt ggcgaacggg      120
   tgagtaatac atcggaacgt gcccgatcgt gggggataac ggagcgaaag ctttgctaat      180
   accgcatacg atctacggat gaaagcaggg gaccgcaagg ccttgcgcgg acggagcggc      240
   cgatggcaga ttaggtagtt ggtgggataa aagcttacca agccgacgat ctgtagctgg      300
   tctgagagga cgaccagcca cactgggact gagacacggc ccagactcct acgggaggca      360
   gcagtgggga attttggaca atgggcgaaa gcctgatcca gccatgccgc gtgcaggatg      420
   aaggccttcg ggttgtaaac tgcttttgta cggaacgaaa agactccttc taataaaggg      480
   ggtccatgac ggtaccgtaa gaataagcac cggctaacta cgtgccagca gccgcggtaa      540
   tacgtagggt gcaagcgtta atcggaatta ctgggcgtaa agcgtgcgca ggcggttata      600
   taagacagat gtgaaatccc cgggctcaac ctgggaactg catttgtgac tgtatagcta      660
   gagtacggta gagggggatg gaattccgcg tgtagcagtg aaatgcgtag atatgcggag      720
   gaacaccgat ggcgaaggca atcccctgga cctgtactga cgctcatgca cgaaagcgtg      780
   gggagcaaac aggattagat accctggtag tccacgccct aaacgatgtc aactggttgt      840
   tgggtcttca ctgactcagt aacgaagcta acgcgtgaag ttgaccgcct ggggagtacg      900
   gccgcaaggt tgaaactcaa aggaattgac ggggacccgc acaagcggtg gatgatgtgg      960
   tttaattcga tgcaacgcga aaaaccttac ccacctttga catgtacgga atcctttaga     1020
   gatagaggag tgctcgaaag agaaccgtaa cacaggtgct gcatggctgt cgtcagctcg     1080
   tgtcgtgaga tgttgggtta agtcccgcaa cgagcgcaac ccttgtcatt agttgctaca     1140
   tttagttggg cactctaatg agactgccgg tgacaaaccg gaggaaggtg gggatgacgt     1200
   caagtcctca tggcccttat aggtggggct acacacgtca tacaatggct ggtacagagg     1260
   gttgccaacc cgcgaggggg agccaatccc ataaagccag tcgtagtccg gatcgcagtc     1320
   tgcaactcga ctgcgtgaag tcggaatcgc tagtaatcgc ggatcagaat gtcgcggtga     1380
   atacgttccc gggtcttgta cacaccgccc gtcacaccat gggagcgggt tctgccagaa     1440
   gtaggtagcc taaccgtaag gagggcgctt accacggcag ggttcgtgac tggggtgaag     1500
   tcgtaacaag gtagccgtat cggaaggtgc ggctggatca cctcctttct ggaaaactgc     1560
   attcaatatt gaacgcccac acttatcggt tgttggaaca agccactga                1609
<210> 5
   <211> 1656
   <212> DNA
   <213> Geobacter
<220>
   <223> gene rdhA
<400> 5
   atgggcgaaa tcaacaggag gaatttttta aaagcctcga tgcttggagc agctgcagct       60
   gctgtagcct cggcatctgc ggtgaagggg gccgttagtc ccttggtggc tgaggctgcg      120
   gacattatgg ctccgatcac ggaaacctca gaatttccat acaaggtgga tgcaaagtac      180
   caacgttaca atagtctgaa gaacttcttt gaaaaggctt tagacccgga agcaaacaag     240
   actcctatta agtttcatta cgacgatgtt tccaaaatca caggcaagaa agatacgggg     300
   aaagacctgc ccatacttaa tgcggaaaga cttgggatca aagggcgtcc ggcgacaaat     360
   gcagaaacag gcgttcttta ctttagtcaa catataggtg ccatgttaac ccagcgccat     420
   aatgaaacag gctggaccgg gttggatgcg gccttgaaag ccggcgcctt tgctgtagat     480
   tttgattatt ccggatttaa ctgcgtaagt ggtggtccgg gatgtgtaat cacgccttat     540
   cccataaatc ctatgaccaa tgaaatagct aatgagcctg tcgtggtacc cggtttgtac     600
   aactgggaca atatcgatgt tgaaagtgtg cgacaacaag gccaacagtg gaaattcaaa     660
   tcaaaggaag aagccagtaa aatggttaaa aaggcagcct gtttcctggg agccgatctg     720
   gccggcatcg caccctatga cgagcgttgg acctattcta cctggggcag gaagattcta     780
   aaaccttgta aaatgcccaa cggcagaacc aaattaatgc catgggattt gccaaagatg     840
   ctgtcaggcg ggggagtaga agttttcgga catgcaaaat ttgaacctga ttgggagaag     900
   tatgcaggtt tcaaaccaaa aagtgtgatt gtcttcgttc ttgaagagga ttacgaagct     960
   atacgcacct caccgtcaat catttccagt gccccggtgg ggaaaggtca ctccaatatg    1020
   ggagaagtag cttacaaaat cgcggttttt ctgaggaagc ttggctacta tgcagcgccg    1080
   tgcggaagcg ataccggact aaatgttcct atggccgttc aggccgggct tggagaagca    1140
   ggcagaaacg gacttttaat tacccagaaa ttcggtccga gaattcgcat cgccaaagtc    1200
   tacaccgacc tgaaacttgc tccggacaag ccgagaaaat tcggggtacg cgagttctgc    1260
   cgcctgtgca aaaaatgtgc ggatgcctgc cccgcccagg ccatctccca tgagaaagac    1320
   cctaaggttc tgcagccaga ggattgtgag gtagccgaaa atccatatac tgaaaaatgg    1380
   cattttgatt ccaaccgttg cggctctttc tgggcctata acggtagtcc ctgcataaat    1440
   tgtgtagctg tatgttcgtg gaacaaagtc gagacctgga accacgatgt ggccagaata    1500
   gccactcgaa taccattgct tcaggatgca gcccgcaagt ttgatgaatg gttcggctat    1560
   aacgggcctg taaaccctga tgaaagactt gaatcgggtt atgttcagaa catggtcaaa    1620
   gacttctgga ataatcctga gtctataaaa caatag                              1656
<210> 6
   <211> 551
   <212> PRT
   <213> Geobacter
<220>
   <223> 1,2-dichloroetane dehalogenase
<400> 6
   Met Gly Glu Ile Asn Arg Arg Asn Phe Leu Lys Ala Ser Met Leu Gly
   1               5                   10                  15
   Ala Ala Ala Ala Ala Val Ala Ser Ala Ser Ala Val Lys Gly Ala Val
               20                  25                  30
   Ser Pro Leu Val Ala Glu Ala Ala Asp Ile Met Ala Pro Ile Thr Glu
           35                  40                  45
   Thr Ser Glu Phe Pro Tyr Lys Val Asp Ala Lys Tyr Gln Arg Tyr Asn
   50 55 60
   Ser Leu Lys Asn Phe Phe Glu Lys Ala Leu Asp Pro Glu Ala Asn Lys
   65 70 75 80
   Thr Pro Ile Lys Phe His Tyr Asp Asp Val Ser Lys Ile Thr Gly Lys
   85 90 95
   Lys Asp Thr Gly Lys Asp Leu Pro Ile Leu Asn Ala Glu Arg Leu Gly
   100 105 110
   Ile Lys Gly Arg Pro Ala Thr Asn Ala Glu Thr Gly Val Leu Tyr Phe
   115 120 125
   Ser Gln His Ile Gly Ala Met Leu Thr Gln Arg His Asn Glu Thr Gly
   130 135 140
   Trp Thr Gly Leu Asp Ala Ala Leu Lys Ala Gly Ala Phe Ala Val Asp
   145 150 155 160
   Phe Asp Tyr Ser Gly Phe Asn Cys Val Ser Gly Gly Pro Gly Cys Val
   165 170 175
   Ile Thr Pro Tyr Pro Ile Asn Pro Met Thr Asn Glu Ile Ala Asn Glu
   180 185 190
   Pro Val Val Val Pro Gly Leu Tyr Asn Trp Asp Asn Ile Asp Val Glu
   195 200 205
   Ser Val Arg Gln Gln Gly Gln Gln Trp Lys Phe Lys Ser Lys Glu Glu
   210 215 220
   Ala Ser Lys Met Val Lys Lys Ala Ala Cys Phe Leu Gly Ala Asp Leu
   225 230 235 240
   Ala Gly Ile Ala Pro Tyr Asp Glu Arg Trp Thr Tyr Ser Thr Trp Gly
   245 250 255
   Arg Lys Ile Leu Lys Pro Cys Lys Met Pro Asn Gly Arg Thr Lys Leu
   260 265 270
   Met Pro Trp Asp Leu Pro Lys Met Leu Ser Gly Gly Gly Val Glu Val
   275 280 285
   Phe Gly His Ala Lys Phe Glu Pro Asp Trp Glu Lys Tyr Ala Gly Phe
   290 295 300
   Lys Pro Lys Ser Val Ile Val Phe Val Leu Glu Glu Asp Tyr Glu Ala
   305 310 315 320
   Ile Arg Thr Ser Pro Ser Ile Ile Ser Ser Ala Pro Val Gly Lys Gly
   325 330 335
   His Ser Asn Met Gly Glu Val Ala Tyr Lys Ile Ala Val Phe Leu Arg
   340 345 350
   Lys Leu Gly Tyr Tyr Ala Ala Pro Cys Gly Ser Asp Thr Gly Leu Asn
   355 360 365
   Val Pro Met Ala Val Gln Ala Gly Leu Gly Glu Ala Gly Arg Asn Gly
   370 375 380
   Leu Leu Ile Thr Gln Lys Phe Gly Pro Arg Ile Arg Ile Ala Lys Val
   385 390 395 400
   Tyr Thr Asp Leu Lys Leu Ala Pro Asp Lys Pro Arg Lys Phe Gly Val
   405 410 415
   Arg Glu Phe Cys Arg Leu Cys Lys Lys Cys Ala Asp Ala Cys Pro Ala
   420 425 430
   Gln Ala Ile Ser His Glu Lys Asp Pro Lys Val Leu Gln Pro Glu Asp
   435 440 445
   Cys Glu Val Ala Glu Asn Pro Tyr Thr Glu Lys Trp His Phe Asp Ser
   450 455 460
   Asn Arg Cys Gly Ser Phe Trp Ala Tyr Asn Gly Ser Pro Cys Ile Asn
   465 470 475 480
   Cys Val Ala Val Cys Ser Trp Asn Lys Val Glu Thr Trp Asn His Asp
   485 490 495
   Val Ala Arg Ile Ala Thr Arg Ile Pro Leu Leu Gln Asp Ala Ala Arg
   500 505 510
   Lys Phe Asp Glu Trp Phe Gly Tyr Asn Gly Pro Val Asn Pro Asp Glu
   515 520 525
   Arg Leu Glu Ser Gly Tyr Val Gln Asn Met Val Lys Asp Phe Trp Asn
   530 535 540
   Asn Pro Glu Ser Ile Lys Gln
   545 550
<210> 7
   <211> 331
   <212> DNA
   <213> Geobacter
<220>
   <223> gene rdhB
<400> 7
   gtgaaaggta ttatgaatat atatgatgtg ctgatttgga tggcactggg tatgattgca 60
   ttgcttatcc aatacggaat ctggcggtat ctgaaaggga agggcaaaga taccatacct 120
   cttcaaatct gcgggttcct cgccaacttt ttctttatat ttgctttggc ctggggatat 180
   tccagctttt cggagcgtga ataccaagct attggcatgg gatttatctt cttcggaggc 240
   acggcactta ttcctgcgat tattacttac aggctggcta accatccggc aaaaaaaatc 300
   agagaaagtt cggattctat ttctgcttag c 331
<210> 8
   <211> 1290
   <212> DNA
   <213> Geobacter
<220>
   <223> gene rdhc
<400> 8
   gctctgaaaa caaagaaaaa taaagcagag ttggaaaata gaagaggatg ggaatactat 60
   tatcaatttt ctttacttct cactgccata atagctattt tatatggtgt gttctgggct 120
   cctcaatcgg ttgattacaa gggaatcatc caaaaaaatg ttctcggcgt tatatccata 180
   gaaaaaatga tggggaatca gcatgcctat aaaatcgata cggcacaggg acgtttctat 240
   gcagtatgtg attcggctat aggatatcaa tccaaagttg aggctatgac tatcgtcaac 300
   gaaaagggcc ttatagagaa agttattatt actaaacagg gagagactcc ggtttttttt 360
   gaaaggctca cagaccaaaa gtattttgac ggctttcaag gtcttgcaat caaggagcct 420
   atttatttgg gaggagcata tggatattcg ggatatctgg gtagtataaa aacaaataat 480
   tatattgacg gggtaacagg ttcaacagtg tcgtcacatg ctgtggctga ggctgtgaac 540
   aaagggaatt cttatttatc agggcaattt tttaacaccc aatgggccaa cccttacgat 600
   ttattccagc ttagctggaa ggatatggcc atgattgcga tgtttttaat tgcgtttgcc 660
   tcagccttta tcaagaagct cgtaaaaata cgtctggcat tcctgctggt tagtgtcgtg 720
   gtgcttggtt ttctcgttaa tcagtttgtg acaggcagct tgcttctttc agcaattacg 780
   ttgcagattc ccaggataac taacctgaaa tggtatgtgc ttatggccgg gtctctcgga 840
   tttattatcc ttttgggaaa aaatctgtat tgcgcttgga tttgtccttt tggagcagta 900
   caggagattc ttaacaaggc tgcagggttc aaatctctga atatttccca gaagactatt 960
   aaaatactca ggctggtggc tccgaccatt ttatgggttg cacttctgct gggcacatta 1020
   ctgggtgact atgggacatt ggattaccag ccttttggcg cgctcttcct gttcaaatcg 1080
   gtatggttaa tgtggttgat gttaccgatc ttcttgttta tgagcttatt tattagccgt 1140
   ttttattgta aattcttctg tccggtagga tttatttaca atttgcttaa ccgttggcga 1200
   aatgaggagg taagaatatg gaaacagaga gtagatcgat tgaaacgcaa aaaaaaggaa 1260
   gaacaagaga cctggtcttc acactcttga 1290
<210> 9
   <211> 987
   <212> DNA
   <213> Geobacter
<220>
   <223> gene rdhT
<400> 9
   gtatcaaata gacataacga gggaggaatc gtgataatga aacagttcga acttggacaa      60
   tataaggctc taaatgtcaa gcgttttgat accacagtcc aagaagagga gattcagcaa     120
   gccttagatt atatcatagg ctcttttgat gaaattgaag aagaaaaaag aaatgagcct     180
   attaaaacaa acgattatgt aatcgtggat attgacggtt ttgaaaagga tgcaacagta     240
   ccggttataa gaaacatcga tacaaaatta atagtcggca gtgaaggtgt atttcgagaa     300
   gttagtgcaa atctcctagg gaagaaaatg ggcgacacag tgacctttga gactgttatt     360
   cagcccgatg ccttagaatt tcagcgttgg tggggaagcg agtttacatt taccgttaag     420
   attaagagcg tttttgtggt gaagaaacca gaattgactg aagaattaat taggaaagtt     480
   gaacctgatc taaaaaacct ggaagactta aaaaatatgc ttgccctaaa aatcactcac     540
   gaaaaagagg ggaaggaacg ggaggcaaat attctcttga tttttcaagc tttagttaaa     600
   caatgtaagt atgaatttga tgaagaagaa ttggattcag ctgctgaaga tttatataag     660
   aaatttactg aagaactcaa aatagttgat gatatggaac ttatggaata tttgatccat     720
   agaaaaataa ccgcagacca attgctggcc gaatgtaagg aagaggcatc cagaaggatt     780
   ctctgggaac ttatgataaa ttctgttatt gagaaagaag agataaatct tactccggat     840
   gaaatcaaat atcttgaaaa gcgaattaac gaaagccgac aaaacggtca actccccgaa     900
   gagtttatgg acatcaattt tttactggca tcgtatttac gaaaaaaaac aatagactat     960
   ttgctaaaaa tcaatttggc aagttaa                                         987